# EUROPEAN PATENT APPLICATION

(11) **EP 2 698 733 A1**
(43) Date of publication of application: **19.02.2014**
(21) Application number: 12770590.3
(22) Date of filing: 09.04.2012
(51) Int. Cl.: G06F 17/50

(54) **USES OF MULTI-DIMENSIONAL MATRIX IN PHARMACEUTICAL MOLECULE DESIGN AND PHARMACEUTICAL MOLECULE DESIGN METHOD**

(30) Priority: 11.04.2011 CN 201110089329
(71) Applicant: Yan, Jingbo, Beijing 100048 (CN); Huo, Xiumin, Beijing 100048 (CN)
(72) Inventor: Yan, Jingbo, Beijing 100048 (CN); Huo, Xiumin, Beijing 100048 (CN)
(74) Representative: Deters, Frank
(86) International application number: PCT/CN2012/000467
(87) International publication number: WO 2012/139421

(57) **Abstract**

The present invention relates to the application of multidimensional matrix for drug design and the methodology for drug design, which for the first time introduces the concept of matrix optimization in mathematics to the design of drugs and the relevant molecules. The present invention uses multidimensional matrix to analyze the permutation and combination of factors that affect the chemical structures and properties of drugs, and classifies and compares the huge amounts of factors need to be considered in the drug discovery according to certain features, thus utilizes fewer number of variables to represent the huge number of variable factors to specifically obtain chemical structures for effective drugs and improves the physicochemical properties of the compounds. By structural comparison of the results with the experimental data of known drugs or compounds in all stages of drug discovery, the present invention further optimizes the molecular chemical structure of drugs and significantly increases the specificity and efficiency of drug design, and significantly increases the efficiency of synthesis.

## Description

### Scope of the Invention

The invention relates to the field of methodologies for drug molecular design. Particularly, it relates to the applications of multidimensional matrix for drug molecule design and the methods for drug molecule design.

### Background of the Invention

Currently, Research and development (R&D) for new drug discovery have been going through the following stages after the last 100 years development, such as: 1) Target Discovery; 2) Target Validation; 3) High Throughput Screening (HST); 4) Hit-to-Lead; 5) Lead Optimization; 6) Clinical Trial, etc. Among them, Target Validation and Drug Molecular Design are generally considered as the technical bottleneck in drug discovery process.

From the end of last century, genomics and proteomics have been extensively making tremendous progress. Wherein, genomics had established around 12,000-15,000 new types of proteins or new mechanisms for developing new drugs. However, since then there new targets or mechanisms have shown little impact on new drug discovery. Currently, drug R&D in global pharmaceutical industry is still mainly focused on 300-500 validated biological targets, at main time, various molecular design technologies related drug screening and synthetic methods have been widely utilized.

High Throughput Screening (HTS) is the widely used for drug screening since it was introduced. A study of numerous databases indicated that there are 15-20 million compounds available for high throughput screening. Although one HTS campaign is able to process 120,000 compounds per day using automation, there are still many limitations which concerning the HTS efficiency: 1) the accuracy of biological target, wherein the biological target is required to be used under automatic process at minimum amount; 2) high resolution detection are required to improve the detection level, such as high quality gene chips; 3) high quality of the compound library, which is usually composed of 3-5 million finely selected compounds, such as the high quality compounds with the drug likeness characterization, the related compounds for the certain drug development projects, etc. Not only the quality and purity of the compounds must be considered, but also more importantly, the structurally diversified factors which representing the chemical space, including compound diversity and drug likeness and drugableness, etc, have to be considered as well.

Effective molecular design and evaluation for compound drug likeness are among the focal points for drug discovery in global pharmaceutical industry. Based on calculation, the number of possible drug-like compounds is a enormous, about 10⁶³, How to find out the structural type of compounds for a certain biological efficacy is the major difficult for this method. How to make the chemical space represented by drug-like compounds efficiently corresponds with the biological consisting with protein targets, and how to increase the novel or privilege types and numbers of the compound structures, are major tasks for drug discovery in chemistry. It could be the limitations that are the efficiency of molecule design and the diversity of building blocks used for new drug discovery, which have become another major bottleneck for drug discovery.

Hit-to-Lead is a main method in drug discovery. It has been introduced into the pharmaceutical R&D in the recent years. In this method, drug-like compound was firstly screened by HST to confirm a group of active compounds (Hit), then the lead compound(Lead). was obtained by evaluation and optimization of the active compounds By screening and optimizing the chemical structures of Ht compounds, the structure of the compound as potential Lead can be effectively and precisely obtained for certain biological target by HST. Usually, it will cost 4-6 years starting from the synthesis, screening, efficacy, pharmacology, optimization of the compound structure. It also requires large amount of work on molecular design and molecular structure comparison (Structure-Structure Comparison---SSC). Thus, the drawbacks for this method are it is not very systematical and need to in cooperate with the concept of molecular design, and so on.

Currently, the optimization of the lead compound is the critical step in drug discovery. It includes the optimization of molecular design and molecular structure comparison in order to obtain the core structure of compound, and structure modification to utilize the following effects: 1) increasing the bioactivity or efficacy to certain target; 2) possessing selectivity while maintaining the bioactivity to the certain target; 3) enhancing the function and activity for certain cell; 4) optimizing the efficacy of compound in *vivo;* 5) modifying the absorption, distribution, metabolism, excretion and toxicity (ADME/T), etc; 6) coordinating and matching the requirements for the compound in preparation, administration, delivery, bioavailability and so on.

However, the present optimization procedure for lead compound is rather mechanized and trivial. It included the structural modification of adjusting the substitution groups, heteroatoms, ring systems, the shape of molecules, etc to make it possessed "drug-likeness". Usually, it needs to modify 1-3 compounds having the core structures, and then study the relationship of the structure and bioactivity (SAR). It normally need above 5000 compounds to optimize the structure of the compound in consideration of both pharmacokinetics and pharmacological side effects. This hindered by the drawbacks such as low efficiency in the area molecular design of compound and not able to fully implement the current databases and related methods for pharmaceutical studies.

Focused library is another method to increase drug screening efficiency recently. This method comprised around 500-2,000 compounds, majorly focused to the special biological target. The molecular design methods include the target orientated, diversity orientated, natural product orientated, and fragment orientated, etc. However, all these designs are only based on the individual factor, and considerate very little to the correlations between all the factors. The designs do not take into account of relatively quantitative and comprehensive comparison to evaluate influence on the drug-likeness of the compound, and do not fully utilize the existed historical and experimental data. Thus, the molecular design of the compound library tends to be unitary, and seriously affect the efficiency of the structural design of the compounds.

Although the global pharmaceutical industry had invested a lot of resources to develop many new technologies, with the aim to improve the efficiency of drug discovery, however, the urgent and unmet needs to solve the technical problems in drug discovery, such as: how to improve the effectiveness, specificity and efficiency in drug design to make it more effective, in simple meaning: practical and convenient; The questions remain, how to compare the structures of the compounds reasonably; how to utilize and consider all kinds of factors that affect the biological activity and physicochemical properties of the drug molecules and their relationships in molecular design; how to comprehensively analyze and evaluate many other factors which will affect the structure and characteristics of the compounds for significantly improving the efficiency of drug molecule design.

### Contents of The Invention

In order to accelerate drug discovery process, in particular, to significantly enhance the efficiency of potential drug design, the inventors established multidimensional matrix as the methodological and technical platform for molecular design. Such platform for the first time implements matrix optimization concept in mathematics into molecular design. By classifying and comparing large amounts of factors in drug discovery according to certain characteristics, it can use fewer variables to represent a huge number of variables to improve the efficiency of molecular design and synthesis in drug discovery.

The concept of molecular design by multidimensional matrix is that any drug molecule is based on the combination of the so-called basic building blocks of chemicals. By classifying 3 million valuable "drug-like" compounds based on 28,000 basic chemical building blocks, and then analyzing their structures and building block distribution, it is clear that basic building blocks build up a drug molecule in the way as the permutation and combination of matrix and multidimensional matrix. Besides, structural classification analysis of the structures of natural product and the compounds of the active ingredients of traditional Chinese medicine indicated that there is a high level of similarities of the combination manner of building blocks for the synthesized compound with the natural product templates. Therefore, during drug molecular design process, it can highly increase the specificity and efficiency of drug molecular design by comparing the historical and experimental databases of the structures of the known compounds.

Multidimensional matrix molecular design platform provide a systematic structure comparison (SSC) and optimization methodology in the matrix mode. This method uses the permutation of multidimensional matrix to analyze the corresponding variables of structural factors and corresponding variables of structural related properties factors. By considering the comparison results of the structure portion with historical and experimental databases, it is able to optimize the representative compound structures, and significantly reduces the number of compounds necessary for consideration and synthesis. It can quickly obtain drug candidates with the desired biological activity or specific drug related activity, thus it can significantly increase the efficiency and effectiveness of molecular design.

The method in the invention using multidimensional matrix and comparing the structure of the desired compound to optimize the structure of the candidate drugs or possible drug molecules, and to complete the molecule design of candidate drug or possible drug molecules. It can also be used to optimize Me-Too or Me-Better type of new drugs, drug scaffold compounds, "drug-like" compounds, compounds needed in Hit-To-Lead and lead processes, etc. It can be used to synthesize the optimized drug candidate by minimum variables and minimum number of compounds. It has a strong specificity for molecular design so that it can significantly improve the efficiency of drug design, drug R&D, and significantly reduce the time and costs in research and development in drug discovery.

To fulfill the goals of the present invention, the present invention provides the following technical solutions.
1. A method for optimizing the molecular structures of drug candidates or possible drug molecules, which comprises the following steps:
   (1) Partition the structures of targeted compounds according to basic building blocks, and assign the corresponding structural parts with uppercase letters of A, B, C, D...Y or Z respectively. Define the modifiable parts of the drug candidates, select the possible variables in the modifiable parts respectively, wherein, the variables of modifiable part A are selected from A1, A2, A3...An, the variables of modifiable part B are selected from B1, B2, B3...Bn, the variables of modifiable part C are selected from C1, C2, C3...Cn, the variables of modifiable part D are selected from D1, D2, D3...Dn ......, the variables of modifiable part Y are selected from Y1, Y2, Y3...Yn, the variables of modifiable part Z are selected from Z1, Z2, Z3...Zn, wherein, n is a natural number;
   (2) Select the variable factors and the variables in reference to the historical and experimental data. The variable factors are represented by lowercase letters of a, b, c, d...y or z, wherein, Variables of variable factor a are selected from a1, a2, a3...an, variables of variable factor b are selected from b1, b2, b3...bn, variables of variable factor c are selected from c1, c2, c3...cn, variables of variable factor d are selected from d1, d2, d3...dn, ......, variables of variable factor y are selected from y1, y2, y3...yn, variables of variable factor y are selected from z1, z2, z3...zn, wherein, n is a natural number;
   (3) By permutation of multidimensional matrix, analyze the corresponding variables of modifiable part A, B, C, D...Y or Z in step (1) and the corresponding variables of variable factor a, b, c, d...y or z in step (2). In reference to the results of structural comparison between the structural parts and historical and experimental databases, select the preferred representative structure type of compounds as A', B', C', D'...Y' or Z', complete the structure design and optimization of drug candidates.

The modifiable part in step (1) is preferred to be determined by comparing with historical and experimental databases.

In the preferred embodiments of the present invention, the methods include the following steps:
(1) Partition the structures of targeted compounds according to basic building blocks;
(2) Define the portions in the molecule of drug candidates that affect biological target orientated bioactivity / cellular activity in reference to the historical and experimental database, and assign them as the not initial or not-to-consider modifiable part;
(3) Analyze the structure of the targeted compound, and confirm the structural portions, define the modifiable parts of the drug candidates. Assign the corresponding structural parts with uppercase letters of A, B, C, D... Y or Z respectively. Select the desired variables in the modifiable parts respectively, wherein, the variables of the modifiable part A are selected from A1, A2, A3...An, the variables of the modifiable part B are selected from B1, B2, B3...Bn, variables of the modifiable part C are selected from C1, C2, C3...Cn, variables of the modifiable part D are selected from D1, D2, D3...Dn ......, variables of the modifiable part Y are selected from Y1, Y2, Y3...Yn, variables of the modifiable part Z are selected from Z1, Z2, Z3...Zn, wherein, n is a natural number;
(4) Select the variable factor and the variables in reference to the historical and experimental data. The variable factors are represented by lowercase letters of a, b, c, d...y or z, wherein, variables of the variable factor a are selected from a1, a2, a3...an, variables of the variable factor b are selected from b1, b2, b3...bn, variables of the variable factor c are selected from c1, c2, c3...cn, variables of the variable factor d are selected from d1, d2, d3...dn, ......, variables of the variable factor y are selected from y1, y2, y3...yn, variables of the variable factor z are selected from z1, z2, z3...zn, wherein, n is a natural number;
(5) By permutation of multidimensional matrix, analyze the corresponding variables of modifiable part A, B, C, D...Y or Z in step (3) and the corresponding variables of the variable factor a, b, c, d...y or z. In reference to the results of structural comparison between the structure parts and historical/experimental data, select the preferred representative structure types of compound as A', B', C', D'...Y' or Z'.

In the preferred embodiments of the present invention, the method includes the following steps:

In the same time when the modifiable parts are defined in step (1) or (3), exclude the modification of the not-to-consider part. Such not-to-consider part is selected from any of the substitution groups on the cyclic structures, the functional groups or structures should not be included in drug-like compounds, or the combination thereof.

In the preferred embodiments of the present invention, the method further includes any of the following steps or all of them:
(6) Analyze the structure of the preferred representative structure of compound A', B', C', D'...Y' or Z' selected in step (3) or (5), and confirm the structure. Determine the desired variables, wherein, variables of the modifiable part A' are selected from A'1, A'2, A'3...A'n, variables of the modifiable part B' are selected from B'1, B'2, B'3...B'n, variables of the modifiable part C' are selected from C'1, C'2, C'3...C'n, variables of the modifiable part D' are selected from D'1, D'2, D'3...D'n......, variables of the modifiable part Y' are selected from Y'1, Y'2, Y'3...Y'n, variables of the modifiable part Z' are selected from Z'1, Z'2, Z'3...Z'n, wherein, n is a natural number;
(7) Select the variable factors and the variables that affect drug candidates in reference to the historical and experimental data. The variable factors are represented by lowercase letters of a', b', c', d'...y' or z', wherein, variables of the variable factor a' are selected from a'1, a'2, a'3...a'n, variables of the variable factor b' are selected from b'1, b'2, b'3...b'n, variables of the variable factor c' are selected from c'1, c'2, c'3...c'n, variables of the variable factor d' are selected from d'1, d'2, d'3...d'n......, variables of the variable factor y' are selected from y'1, y'2, y'3...y'n, variables of the variable factor z' are selected from z'1, z'2, z'3...z'n, wherein, n is a natural number;
(8) By permutation of multidimensional matrix, analyze the corresponding variables in the preferred representative compound structure of A', B', C', D'...Y' or Z' from step (6) and the corresponding variables of the variable factor a', b', c', d'...y' or z' from step (7). By referring to the results of structural comparison between the structure parts and historical/experimental data, select the preferred compound structure of A'B', B'C', C'D'...Y'Z'; or
(9) According to the requirements, based on the methods in step (6)-(8), by permutation analysis of multidimensional matrix, select the corresponding variables in the preferred representative compound structure of A'B', B'C', C'D'...Y'Z' and the corresponding variables of variable factor a'b', b'c', c'd'...y'z'. By referring to the results of structural comparison between the structure parts and historical/experimental data, select the preferred representative compound structure of A"B"C", B"C"D"...X"Y"Z"; or
(10) According to the requirements, based on the methods in step (6)-(8), by permutation analysis of multidimensional matrix, select the corresponding variables in the preferred representative compound structure of A"B"C", B"C"D" ...X"Y"Z" and the variable factor of a"b"c", b"c"d"...x"y"z". By referring to the results of structural comparison between the structure parts and historical/experimental data sequences, complete the structure design and optimization of the drug candidates; or
(11) Optionally, according to the requirements of the design for drug candidates, repeat part of the mentioned steps above or all the steps by multidimensional matrix, analyze the structures, confirm the structures and optimize the structures of the drug candidates until to obtain the desired structures of drug candidates.

In the preferred embodiments of the present invention, the building blocks comprise any structure unit in a molecular structure, which is selected from any of saturated or unsaturated mono-cyclic structure unit, bi-cyclic structure unit, multi-cyclic structure unit, substitution group, functional group or the combination thereof;

In the preferred embodiment of the present invention, said mono-cyclic structure unit is selected from any mono-cyclic aromatic ring, mono-cyclic non-aromatic ring, substituted mono-cyclic aromatic ring, substituted mono-cyclic non-aromatic ring or the combination thereof;

In the preferred embodiment of the present invention, said bi-cyclic structure unit is selected from any bi-cyclic aromatic ring, bi-cyclic non-aromatic ring, substituted bi-cyclic aromatic ring, substituted bi-cyclic non-aromatic ring or the combination thereof;

In the preferred embodiment of the present invention, said multi-cyclic structure is selected from any multi-cyclic aromatic ring, multi-cyclic non-aromatic ring, substituted multi-cyclic aromatic ring, substituted multi-cyclic non-aromatic ring or the combination thereof, wherein, the number of rings is not less than 3;

In the preferred embodiment of the present invention, said functional group is selected from any ketone, aldehyde, ester, amine, amide, single bond, double bond, triple bond, halogen, acid, alcohol, thiol, sulfonic acid, phenol, thiophenol or the combination thereof;

In the preferred embodiment of the present invention, said substitution group is a structural moiety of any compound, which is selected from any alkyl group, alkenyl group, alkynyl group, hydroxyl group, ether group, ester group, aryl group, heteroaryl group, cycloalkyl group, heterocyclic group or the combination thereof.

In the preferred embodiment of the present invention, said modifiable part is the structure part affect bioactivity or cell specificity of the compound.

In the preferred embodiment of the present invention, said historical/experimental data are selected from any of the biological target bioactivity, the biological target selectivity, cell activity, toxicity and side effects, ADME properties, drug likeness, synthesizability or the combination thereof.

In the preferred embodiment of the present invention, according to the requirements of the design for drug candidates, steps (1)-(8) can be repeated partially or entirely by multidimensional matrix, to analyze the structure, confirm the structure and optimize the structure of the drug candidates until to obtain the structure of drug candidates for the desired bioactivity or pharmacological activities.

In the preferred embodiment of the present invention, said historical and experimental data are selected from any of the following databases or the combination thereof:
1) Databases of protein target commonly used in world drug discovery field and the databases of the corresponding compound structures; or
2) Databases of the structure types of the corresponding compounds for the protein targets commonly used in world drug discovery; or
3) Databases of core structures of compounds for drug discovery; or
4) Databases of the framework compounds for drug molecules; or
5) Databases of the structures of the verified bioactive compounds; or
6) Databases of the queryable marketed drugs; or
7) Databases of bioequivalence and bioisoterics; or
8) Databases of the metabolic compounds; or
9) Databases of the structures of the toxic compounds; or
10) Databases of the active ingredient compounds in Chinese medicine; or
11) Databases of the monomeric compound structures of natural products; or
12) Database of therapeutics; or
13) Database of medical keywords.

The aim of the present invention is to provide the application of multidimensional matrix for drug molecule design, wherein, the permutation of said multidimensional matrix is determined jointly by structural factors and experimental data.

Preferentially, by permutation of multidimensional matrix, analyze the corresponding variables in structural factors and variables in variable factors in compounds. By referring to the results of structural comparison between the structure parts and historical/experimental data, select the preferred representative structure of the compound.

In the preferred embodiment of the present invention, said drug molecule is selected from any of Me-Too or Me-Better type new drugs, drug scaffold compounds, "drug-like" compound, compounds used in Hit-To-Lead, lead optimization processes or the combination thereof.

Preferentially, use any mentioned methods above of drug molecule design to design drug molecule in the application of drug molecule design.

### DETAILED DESCRIPTION OF THE INVENTION

In order to clarify the protection scopes of the present invention, the terms in the present invention are explained as following

Said building blocks in the present invention comprise any structure unit in a molecule, which are selected from any saturated or unsaturated mono-cyclic, bi-cyclic ring, multi-cyclic ring structure units, with any substituted group, functional group or the combination thereof; said mono-cyclic structure unit is selected from any mono-cyclic aromatic ring, mono-cyclic non-aromatic ring, substituted mono-cyclic aromatic ring, substituted mono-cyclic non-aromatic ring or the combination thereof; said bi-cyclic structure unit is selected from any bi-cyclic aromatic ring, bi-cyclic non-aromatic ring, substituted bi-cyclic aromatic ring, substituted bi-cyclic non-aromatic ring or the combination thereof; said multi-cyclic structure is selected from any multi-cyclic aromatic ring, multi-cyclic non-aromatic ring, substituted multi-cyclic aromatic ring, substituted multi-cyclic non-aromatic ring or the combination thereof, wherein, the number of rings is not less than 3; said functional group comprise any ketone, aldehyde, ester, amine, amide, single bond, double bond, triple bond, halogen, acid, alcohol, thiol, sulfonic acid, phenol, thiophenol or the combination thereof; said substitution group is a structural moiety of any compound comprising any alkyl group, alkenyl group, alkynyl group, hydroxyl group, ether group, ester group, aryl group, heteroaryl group, cycloalkyl group, heterocyclic group or the combination thereof.

Currently, there are 30,000 basic structural types, functional groups and elements in chemical space of drug discovery. By multidimensional matrix in the present invention, the basic structure type can be determined as about 500, the commonly used functional groups are determined as 30-50.

Said "ADME/T" in the present invention refers to the properties of compounds in absorption, distribution, metabolism, excretion and toxicity.

Said modifiable part in the present invention refers to structure part of compound that affects bioactivity and cell specificity.

Said un-modifiable part in the present invention refers to the structure part that determine the bioactivity or cell activity of the compound and can not be alternated or modified rashly.

Said "not-to-consider part" in the present invention refers to the factors or variables to be considered in the later stage of drug design, which comprise substitution of cyclic structure, this part is concerned with certain properties of the compound but belongs to the additional part of drug compound, and considered as more optional variables. It has less effect to the variable factors of drug candidates, normally it should be considered with basic cyclic system connected to it altogether. Thus, it can be considered in the late stage to efficiently decrease the variable factors in compound design and increase the design efficiency significantly.

Said target or biological target in the present invention refers to protein has certain effects to a given indication for diseases. It can be classified according to its biological effects, indications (such as antitumor, heart disease, central nervous system diseases, etc.), target type (such as GPCR, ion channels etc.). Meanwhile, any biological target or protein can contains the target point, the same target corresponds to different target point and correspond to different bioactivity or indication and has different effects. The same target point only has the efficient activity to one biological activity or indication.

Said "target or targeted compound" in the present invention can be considered as "reference compound", "target for drug design" or "reference", which comprise the known structure of the compound have certain bioactivity to specific biological target and target point, i.e., so called the known compound structures.

Said "known compound structure" in the present invention refers to the structure of the compound disclosed in patents or scientific literatures that has bioactivity to certain biological target, which comprises compounds as the marketed drugs, drug candidates in the reporting stage or clinical stage, and pre-clinical stage.

In the preferred embodiment of the present invention, the way that target compound is selected comprise indication, corresponding target of indication, verified target or well-accepted target or target, target group or protein group (such as GPCR, ion channels, etc.) with clear mechanism, the structure of target protein, structure of compound that are disclosed in patents or scientific literatures.

In the preferred embodiment of the present invention, said target compound is selected from any known compound structure with certain bioactivity, the inquired compound structure according to the code of target database or compound structure has certain effects to target, compound structures of the known drugs or drug candidates etc., which comprise the marketed drugs, drug candidates in clinical stages, and pre-clinical stages, lead compounds, natural products possessing bioactivity, mono compounds in Chinese medicine, active ingredients of Chinese medicine, compound with verified bioactivity from drug-like compounds, compounds from computer-aided drug design (CADD designed compound), compounds of high throughput screening, known stereo-structure of target proteins or the target parts or the combination thereof.

Drug molecule design in reference to the target compound is the major direction for R&D for new drugs. It is to analyze, design, modify and optimize the compound structure of the designed compounds regarding to the target, to obtain new compound structure or lead compound structure, and it can be used to validate biological target, and find or design new structure of drug compound (such as Me-Too or Me-Better drug), and so on.

Said compound structure in the present invention refers to compounds have similar structure and bioactivity to specific biological target.

Said drug candidates in the present invention refers to new compound structure (new chemical entity, NCE) has the potency to be able to develop into marketed drug.

Said "analyze, confirm and optimize the compound structure" in the present invention refers to analyze any factors that affect the drug candidates to be a drug or the combination thereof by permutation of multidimensional matrix, to use the minimum number of the consideration factors to design drug molecule efficiently, to obtain the compound structure of the optimized lead compound or drug candidates.

Said target bioactivity in the present invention refers to the bioactivity or cell activity to a certain biological target of the compound.

Said target biological selectivity in the present invention refers to the selectivity of the compound to the different target points in biological targets.

Said cell activity in the present invention refers to the bioactivity to certain cells.

Said toxicity and side effect in the present invention refers to the toxic and/or side effect of the compound.

Said synthesizability in the present invention refers to the possibility that the compound can be synthesized.

Said "optimization of lead compound" in the present invention refers to optimizing the structures and properties of the compound with certain bioactivity, to obtain drug candidates with the desired bioactivity or cell activity.

Currently, cheminformatics was used to define the "drug likeness", which uses some summarized physicochemical parameters to determine the "drug likeness" of a compound and increase the design rate of the active compound (Hit) and lead compound (Lead), wherein, the parameters to determine "drug likeness" of a compound are coming from known drugs, drug candidates in clinic stages, analysis and identification results of natural products.

Said "drug likeness" (drug like) compound in the present invention has its meaning comes from Walters and Murcko (Walters WP, Stahl MT, and Murcko MA. Virtual Screening: An overview. Drug Discovery Today 1998; 3:160-78; Walters WP, Murcko A, Murcko MA. Recognizing Molecules with drug-like properties. Curr Opin Chem Biol 1999; 3:384-7). Based on their studies to the listed drugs in United States Pharmacopoeia, they pointed out the molecule structures of "drug likeness" compounds should be in consistence with the functional groups and physicochemical properties in the majority of the known drugs. The properties of current "drug like" compound come from the studies and summary of the known drugs, but the known drugs only cover a minor portion of "drug like" compound, which can not represent all kinds of "drug like" compounds. Lipinski (C.A. Lipinski; F. Lombardo; B.W. Dominy and P.J. Feeney (1997). "Experimental and computational approaches to estimate solubility and permeability in drug discovery and development settings". Adv Drug Del Rev 23: 3-25.; Lipinski CA, Lombardo F, Dominy BW, Feeney PJ. Experimental and computational approaches to estimate solubility and permeability in drug discovery and development settings. Adv Drug Deliv Rev 2001; 46:3-26) pointed out "drug like" compounds should have enough acceptable ADME/T (absorption, distribution, metabolism, excretion and toxicity) properties, and should pass clinic trials phase I. They distributed in a vast chemical space, which comprise about 10⁴⁰-10¹⁰⁰ "drug like" compounds, comparing to the possible biological targets, the possibility to find out an active compound is less than 1/10¹⁴. The physical properties of the "drug like" compounds could majorly determine how much a compound can be an active compound. Lipinski did not only invent the famous "5 Principles" to help to identify and analyze "drug like" compounds, but also point out ADME/T properties of drugs should be considered starting at High Throughput Screening stage. This is different to the conventional protocol that ADME/T properties are only considered in the later stage of compound optimization.

Current commercial compound databases comprise such as the following, but not limited:
1) Comprehensive Medicinal Chemistry (CMC);
2) World Drug Index (WDI);
3) MDDR database;
4) Investigational Drug Database (IDDB);
5) Available Compound Database (ACD/SCD);
6) ChemNavigator
7) Biologically Active Natural Products (BDNP)

Therefore, those technical personals in drug discovery area could use the databases to obtain the target compounds.

In the preferred embodiment of the present invention, said target compound is the known drug structure. Preferentially it is the widely used drugs in the market, such as anti-diabetic drugs, cardiovascular drugs, and so on.

The present invention uses the clinically broadly verified compound structures for drug discovery, and optimizes and modifies the structures regarding to the new biological targets, to design new compound structures for drugs for certain indication, including the lead compounds.

Said "experimental data/historical data" in the present invention is also "empirical parameters" or "experimental parameters", refers to the data accumulated during drug discovery history and experimentally verified. Said empirical data is selected from target bioactivity, target bioselectivity, cell activity, toxic side effects, ADME properties, drug likeness, synthesizability or pharmacokinetics & pharmacology parameters, etc. These experimental data have close connections to the compound structures, including the structure-activity relationship of the compounds. Thus, the process of comparison of experimental data includes the comparison of the compound structure and the compound optimization.

The experimental data in the present invention are all the known databases, for examples:
1) The protein target databases and the corresponding compound structure databases that are commonly used in world drug discovery field. Compound databases in the clinical stages, related information of compounds in pre-clinical stages, and the information of protein targets related to structures, including the target discovery, the target validation, protein structures and the related compound structures. The representative databases comprises:
   http://thomsonscientific.ip/products/iddb/index.shtml;
   http://www.cancer.gov/cancertopics/factsheet/Therapy/investigational-drug-access;
   http://science.thomsonreuters.com/support/faq/sddb/;
   http://www.centerwatch.com/drug-information/pipeline/;
   http://www.pharmaprojects.com/research_development_analysis/tools.htm;
   http://www.pipelinereview.com/store/product_info.php?products_id=2741;
   http://www.bioportfolio.com/store/product/7781/R-d-Drug-Pipeline-Database-2-mont hs-Subscription.html;
   http://thomsonreuters.com/products_services/science/science_products/a-z/pipeline_d ata_integrator/;
   http://www.ovid.com/site/catalog/DataBase/1244.jsp:
   http://www.imshealth.com/portal/site/imshealth;
   http://www.pjbpubs.com/; or
   http://www.fda.gov/.

ADME databases for studying and summarizing properties of the absorption, distribution, metabolism and excretion of compounds, wherein the representative databases comprise:
http://www.pharmainformatic.com/html/adme_tox_redictions.html;
http://www.aureus-sciences.com/aureus/web/guest/adme-overview;
http://jp.fujitsu.com/group/kyushu/services/lifescience/english/asp/admedb/;
https://www.cloegateway.com/services/cloe_knowledge/pages/service_frontpage.php;
http://www.siritech.com/Cheminformatics.htm;
http://modem.ucsd.edu/adme/databases/databases_extend.htm;
http://www.pubpk.org/index.php?title=Main_Page;
http://www.pubpk.org/index.php?title=Main_Page;
http://www.hmdb.ca/;
http://www.nugo.org/metabolomics/36124;
http://www.genome.jp/kegg/pathway.html;
http://kanaya.naist.jp/KNApSAcK/;
http://accelrys.com/products/databases/bioactivity/metabolite.html; and
http://metlin.scripps.edu/.

Protein target databases for seeking the information of protein target related to the diseases, which comprise the target discovery, the target validation, protein structures and the corresponding compound structures. The representative databases comprise:
http://targetdb.pdb.org/
http://www.dddc.ac.cn/pdtd/
http://www.rcsb.org/pdb/home/home.do
http://bidd.nus.edu.sg/group/CJTTD/TTD.asp
http://www.sciclips.com/sciclips/drug-targets-main.do
http://www.ncbi.nlm.nih.gov/genbank/
http://www.ebi.ac.uk/Databases/structure.html

Databases of the methodology for compound syntheses for seeking the synthetic methods and the synthesizabilities, wherein representative databases comprise:
https://scifinder.cas.org;
http://accelrys.com/products/databases/synthesis/; and
http://www.thieme-chemistry.com/en/products/journals/synfacts.html.

Databases of natural product and Chinese traditional medicine for searching compound structural data of natural products and Chinese traditional medicine, wherein the representative databases comprise:
http://naturaldatabase.therapeuticresearch.com/home.aspx?cs=&s=ND;
http://www.ponderfodder.com/node/113;
http://www.ncbi.nlm.nih.gov/pmc/articles/PMC1347494/;
http://dnp.chemnetbase.com/intro/index.jsp;jsessionid=80C9568C977F47200197BE4 8213AC51A;
http://www.heterocycles.jp/structure/structure.php;http://www.chemnetbase.com/;
http://www.gfmer.ch/TMCAM/TNCAM_database_system.htm;
http://www.rmhiherbal.org/ai/pharintro.html;
http://tcm.cmu.edu.tw/about01.php?menuid=1; and
http://tcm.cz3.nus.edu.sg/group/TCMsite/Default.aspx

Databases of "drug like" compounds, bioactivitive compounds for looking for information of "drug like" compound s and bioactivitive compound s, wherein the representative databases comprise:
http://accelrys.com/products/databases/bioactivity/mddr.html;
http://accelrys.com/products/databases/bioactivity/comprehensive-medicinal-chemistr y.html;
http://www.chemnavigator.com/; and
http://accelrys.com/products/databases/sourcing/screening-compounds-directory.html.

Databases of drug toxic side effects for seeking the toxicities and side effects properties of compounds, wherein the representative databases comprise:
http://databases.biomedcentral.com/browsesubject/?sub_id=1013;
http://www.drugs.com/;http://sideeffects.embl.de/;
http://www.pdrhealth.com/drugs/drugs-index.aspx;
http://www.drugs.com/drug_interactions.html;
http://www.pdrhealth.com/home/home.aspx;
http://www.rphworld.com/link-350.html;http://toxnet.nlm.nih.gov/;
http://bioinf.xmu.edu.cn/databases/ADR/index.html;
http://ctd.mdibl.org/; and
http://accelrys.com/products/databases/bioactivity/toxicity.html.

Databases for the known drugs, which can provide the basic information for drugs including the mechanism for protein targets, molecular structures of drugs, pharmacokinetics & pharmacology properties, toxicities and side effects, drug-drug interactions, etc. wherein the representative databases comprise:
http://www.drugbank.ca/;http://www.nlm.nih.gov/medlineplus/druginformation.html;
http://chrom.tutms.tut.ac.jp/JINNO/DRUGDATA/00database.html;
http://www.rxlist.com/script/main/hp.asp;
http://www.accessdata.fda.gov/scripts/cder/drugsatfda/;
http://www.fda.gov/Drugs/InformationOnDrugs/ucm142438.htm;
http://www.ncbi.nlm.nih.gov/pubmed/;http://www.webmd.com/;
http://www.3dchem.com/atoz.asp;http://www.drugs.com/; and
http://www.pdrhealth.com/home/home.aspx.

In the preferred embodiment of the present invention, when design drug candidates by multidimensional matrix, the first step is to confirm the structure of the target compound, that is, partition the molecular structure of the compound according to the building blocks. Then in reference to the experimental data, conduct comparative analysis and structural optimization to use the minimum number of variable parts or the modifiable parts.

The compound structures interacted with biological targets mostly have certain core structure, such structural core reflects the bioactivity of the compound to the specific target, wherein, the stereo configuration of the structural core should match the stereo configuration of target protein packet. The matching degree between them is the major factor to determine the bioactivity of such compound. The distribution of the hetero atoms in the structural core of the compound is correlated to the bioselectivity. The distribution of the functional groups in the structural core is correlated to the selectivity of its bioactivity, and any distributions of hetero atoms and functional groups in the compound structure could all have effects to the pharmacokinetics, pharmacological and toxic side effects, etc. of the compound.

Moreover, different core structures have bioactivity to certain biological targets, the determination factor is the molecular stereo configurations of the compound and the protein. Therefore, in the process of structure design for the compound, it is necessary to compare molecular structures. It can extend the compound structure comparison scope by chemical genetic engineering techniques, and increase the consideration factors to further validate the biological targets, and find the new types of lead compound structures.

There are many factors that must be considered in drug molecular design, such factors includes any of indication, bioactivity, synthesizability, physicochemical properties, stability, metabolism, pharmacokinetics, pharmacology, toxic and side effects or the combination thereof. How to efficiently evaluate and analyze the related affecting factors is the main task in drug molecular design. The affecting factors or the sequences to be considered are different when design different objectives, which are required to consider the factors repeatedly in some cases.

When drug candidates is designed based on a target compound, it is better not to focus on changing or increasing the bioactivity and the selectivity, and focus on improving or increasing its pharmacokinetics & pharmacology properties and decreasing the toxicities and side effects by rational structure modification, wherein, the design need to consider the following factors:
A. protein target (also known as "biological target"); or
B. validation status of target; or
C. compound structures for specific target; or
D. verified "drug like" compound with structure bioactivity; or
E. compound structures of known drugs; or
F. compound structures of drug candidates in clinical research stage; or
G. compound structures of drug candidates before the clinical research stage; or
H. compound structure of natural product; or
I. compound structure of active ingredients of Chinese medicine; or
J. compound structure of bioequivalence; or
K. structure of metabolism product and/or intermediate; or
L. structure of pharmacokinetics pharmacological molecule; or
M. toxic compound structure; or
N. basic building blocks for compound; or
O. basic structures of functional groups of compound; or
P. any synthesized structure or the combination thereof.

In the preferred embodiment of the present invention, when it is required to maintain and improve the bioactivity and selectivity of the compound, the factors to be considered are selected from any of A, B, C, D, E, F, G, H, I, K, P or the combination thereof.

In the preferred embodiment of the present invention, when it is required to maintain and improve the stereo-structure of the compound, the factors needed to be considered are selected from any of A, D, E, H, I, N, O, P or the combination thereof.

In the preferred embodiment of the present invention, when it is required to maintain and improve the metabolism of compound, the factors needed to be considered are selected from any of E, F, H, I, K, N, O, P or the combination thereof.

In the preferred embodiment of the present invention, when it is required to maintain and improve the pharmacokinetics and pharmacology properties of the compound, the factors needed to be considered are selected from any of D, E, F, G, H, I, L, P or the combination thereof.

In the preferred embodiment of the present invention, when it is required to decrease the toxic side effects of the compound, the factors needed to be considered are selected from any of E, F, G, H, I, L, M, P or the combination thereof.

In the design of compound molecular structure by multidimensional matrix, any of factors A-P can be taken into account alone or the combination thereof, and can be considered, which is aimed to combine different factors efficiently and determine the structure of the target compound. Compound structures can be analyzed by methods of multidimensional matrix.

In the preferred embodiment of the present invention, said target is 12,000-15,000 targets obtained from Genebank, Target DB, Threapuetic Target DB, DART, PDTD, TRMP, and other relevant databases, etc. It comprise the validated targets, widely utilized targets, etc. to determine the corresponding compound structures, and design new compound structure types for drugs, new types of lead compounds, and so on.

In the preferred embodiment of the present invention, said target compounds are selected from compound structures of natural products or the active ingredients of Chinese traditional medicine. It can be combined with its characters as traditional medicine and conduct structural comparisons with the structures of the protein target and optimize the structures to find out the efficient new compound structures or lead compound structures. Wherein, said natural products are obtained from databases as the Directory of Natural Product, Traditional Chinese Medicine Database, Natural Product Database, etc.

In the preferred embodiment of the present invention, analyzing and comparing the structures of active compound structure and the biological targets to find out new active compound structures corresponding to the biological target. Wherein, said active compound is the verified compound structure types having certain bioactivity, and represent the maximum number of compound structures in the chemical space, which comprise compound structures of natural products, the known, inquired and obtained from literatures and relevant databases (including PubMed, CMC, MDDR, IDDB, Scifinder, Chemnivagator, etc.), etc..

Compared to the current methodologies, the advantages of the present invention comprise:
1, Multidimensional matrix is systematically used to analyze, design and optimize molecular structure of the compound in the present invention to significantly and precisely improve the comprehensiveness, specificity, accuracy, systematic and design effectiveness.
2, In the present invention, by the combining with efficient drug molecular design, and utilizing information of the known drugs and/or related compounds to verify the design method for drug candidates, it significantly deepens the knowledge and understanding of the relationships between molecular structure of drug candidates and the relevant properties, which further improves and systematizes the statistics efficiency of structure-activity relationships (SAR), and dramatically decreases the cost for drug R&D.
3, The present invention utilizes and summarizes experimental or historical data for drug discovery systematically, comprehensively and rationally. It significantly increases the specificity, efficiency and effectiveness of drug molecular design by systematically and fundamentally improving the design, structure comparison, structure confirmation and optimization of drug candidates.

### DESCRIPTION OF THE FIGURES

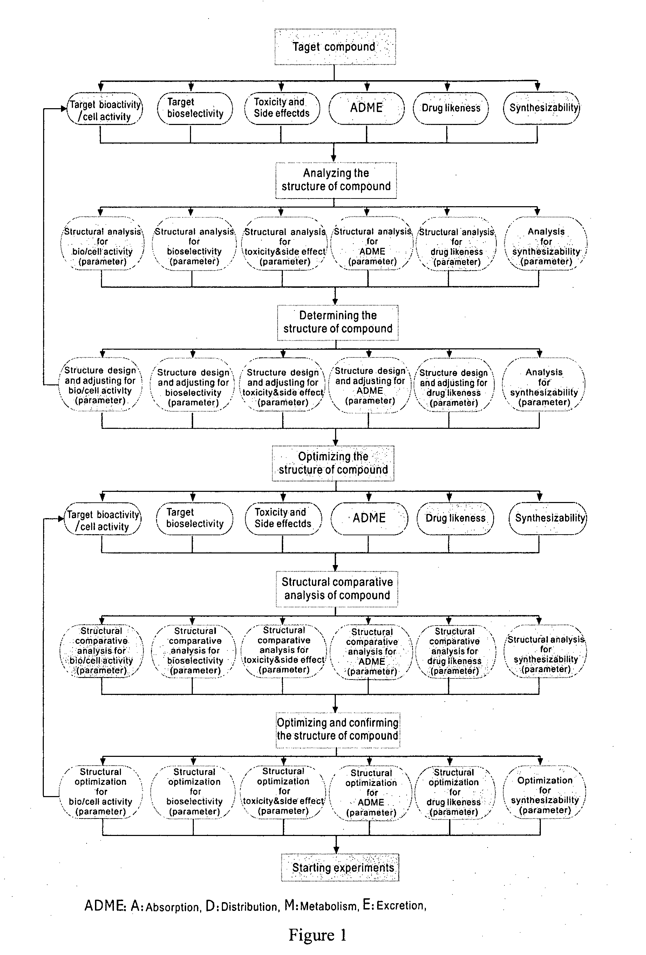

Figure 1: Scheme of optimizing molecular design of the target compound in the present invention.
Figure 2: An example of the multidimensional matrix for optimizing the molecular design of compound in the present invention.

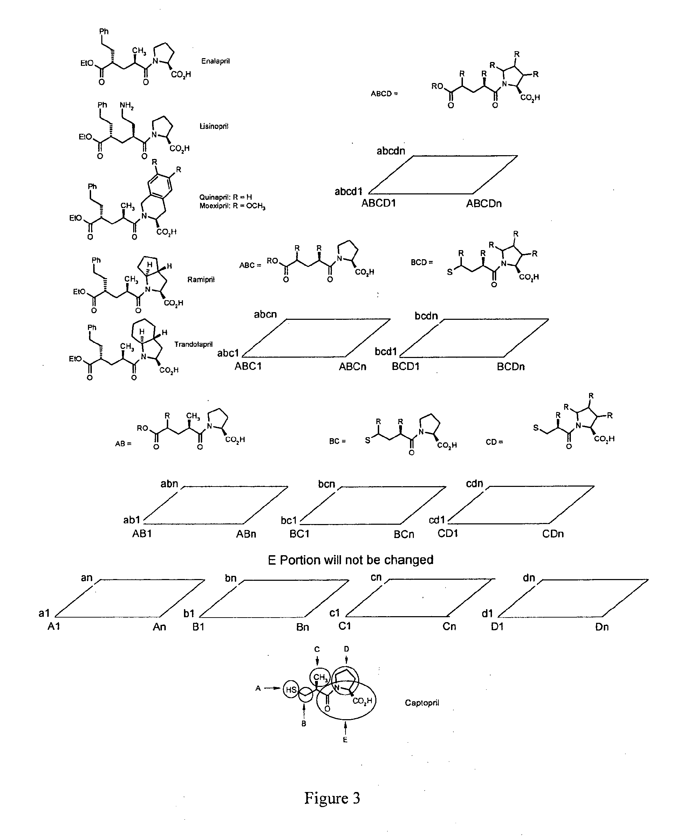

Figure 3: Scheme of the optimization of target compound captopril in Example 1.

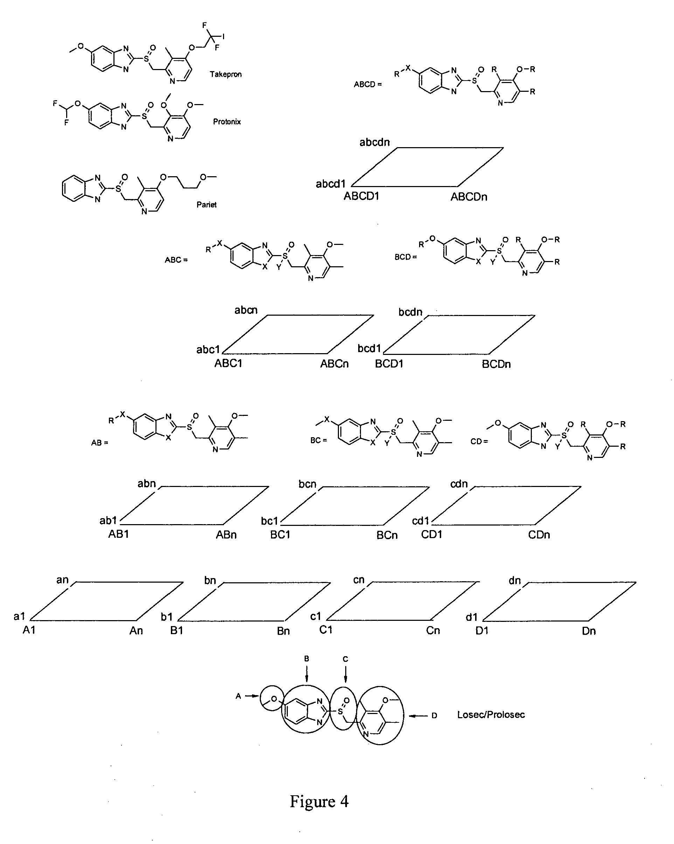

Figure 4 : Scheme of the optimization of target compound omeprazole in Example 5.

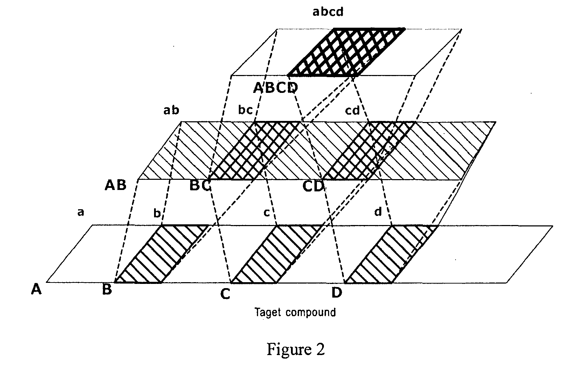

In Figure 2, uppercase letters of A, B, C......Y or Z; AB, AC......BC, BD......CD, CE......XY or YZ represent the sequences of the structural parts in the compounds. Lowercase letters of a, b, c......y or z; ab, ac.....bc, bd......cd, ce......yz represent the sequences of experimental or historical data.

### EXAMPLES

The following examples are further descriptions for the present invention. It should be understood that these examples are not the limitations to the scope of the present invention. Any modification based on the present invention is not beyond the spirits of the present invention.

It should be understood that the hydrogen atom in the following compounds is not shown completely.

### Example 1 Structural optimization of series of compounds with captopril type

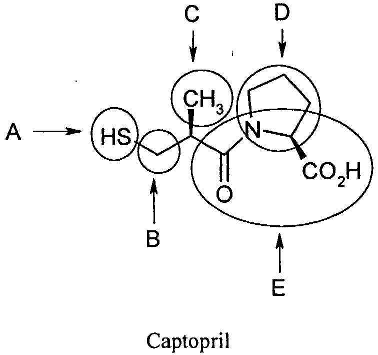

A method of drug molecular design using captopril as the target compound, with the particular steps as following:
1) Partition the structure of captopril according to the building blocks, which resulted to five parts as A, B, C, D, E;
2) In reference to and comparison with the experimental/historical data, part E was defined as the key core part of the structure. Wherein, the amide group, the neighbor acid group and the heterocyclic ring belong to core structure that must be kept. In consideration of the function that it determined the bioactivity/cell activity to the target, part E was confirmed as un-modifiable part, and A, B, C, D are the modifiable parts in molecular design;
3) According to the effects to drug candidates from the variable factors, it could be used for confirming the modifiable part for drug candidates. The particular steps are listed as followings:
   ① Part A. It could be known in reference to and comparison with the experimental/hostorical data that thiol group (SH) functional group has strong reductive property, which is not a suitable functional group for metabolism, formulation type, stability, toxicity and side effects, etc. It can be replaced by OH, NHR, NH₂, SOR, SO₂R, SO₃H, SO₃R, COOH, COOR or heterocyclic building blocks, etc.
   ② Part B. In reference to and comparison with the experimental/hostorical data, to increase the length of the carbon chain and to substitute the elements are the options needed to be considered. The substitution for the elements comprises: O, N, S and heterocyclic building blocks.
   ③ Part C: In reference to and comparison with the experimental/historical data, the stereo configuration must be kept. The stereochemical configuration needs to be kept, and the substitution for methyl group can increase the "drug likeness" of the compound, particularly for pharmacokinetics & pharmacology. The functional groups for substitution are: any aromatic or non-aromatic functional groups.
   ④ Part D: In reference to and comparison with the experimental/historical data, the stereochemistry must be kept, and different cyclic structures and bi-cyclic structures need to be considered. The mono-cyclic (including heterocyclic) ring structure for consideration comprise: 4 numbered ring, 5 numbered ring, 6 numbered ring, 7 member ring, 8 member ring or their heterocyclic rings, etc., The bi-cyclic (including heterocyclic) structure for consideration comprise: 4-5 type, 5-5 type, 5-6 type, 5-7 type, 5-8 type, 6-5 type, 6-6 type, 6-7 type, 6-8 type or their heterocyclic rings.

   According to the analysis of experimental/historical data, 5-6 type is the optimum. In consideration of variable factors such as pharmacokinetics & pharmacology, drug likeness, structure comparison with the known drug, structure comparison with natural products, equivalences, etc., the selection of 5-6 type and 6-6 type is most rational, wherein, the major options are non-aromatic rings, and then the aromatic ring or non-aromatic ring.
4) According to the experimental/historical data, select the variable factors which could affect drugs and their variables, in particularly the steps are as following:
   For part A, the experimental/historical data needed to be considered is in range of target bioselectivity, toxicities and side effects, ADME properties, drug likeness, synthesizability.

The major scope in the consideration needs to be according to the supports of experimental/historical databases, includes bio-equivalence, metabolism databases, "drug like" compound databases, the known drug databases, clinic drug databases, etc. According to the characteristics of SH group, it can confirm that the functional groups for experimental/historical parameters a can be: SOR, SO₂R, SO₃H, COOH, COOR and rings like building blocks. Wherein, SOR has problems in stability; SO₃H and COOH as the strong acidic functional group, have problems in "drug likeness" and the structure comparison with the known drugs; commonly used solution for pharmacokinetics & pharmacology is SO₃R to adjust the structure factor, but SO₃R has the same chemical instability problem as it can be converted to be SO₃H under acidic condition; COOR possesses certain chemical stability, and can be the best option. According to structures and properties of natural products, this part can use ring systems to reduce the number of the rotatable chemical bonds. The optional ring type is 6-8 numbered ring.

For part B, the experimental/historical data parameter b needed to be considered is in the range of target bioselectivity, ADME properties, synthesizability.

Combination of functional groups of O, N, S, etc. in part B and COOR could form urea like compound structure type, which does not fully fulfill the structure of "drug like" compound. Elongation of the carbon chain could not only satisfy the requirements for stability, but also has advantages in adjusting pharmacokinetics & pharmacology of compound. The increased carbon chain number should be 1-2 carbon to fulfill the stereo configuration of the compound.

For part C, the experimental data parameter c need to be considered is in range of target bioselectivity, ADME properties, synthesizability.

For part D, the experimental data parameter d need to be considered is in range of target bioselectivity, toxic side effects, ADME properties, synthesizability.
5) According to the results of structure comparison of each structure part and experimental data sequence, the representative compound structure type was selected out, as the following:
   By comparative analysis of matrix Aa, it was confirmed that the typical structural type of part A was COOR or 6-8 member ring structures.

By comparative analysis of matrix Bb, it was confirmed that the typical structural type of part B was carbon chain structures.

By comparative analysis of matrix Cc, it was confirmed that the typical structural type of part C was any aromatic or non-aromatic functional groups.

By comparative analysis of matrix Dd, it was confirmed that the typical structural type of part D was mono-cyclic ring or bi-cyclic structures.
6) According to the results of comparative analysis of structures of experimental/hiatorical data, the secondary consideration of multidimensional matrix was the following:
   For combination AB, the experimental/historical data, parameters ab need to be considered is in range of target bioselectivity, toxic side effects, ADME properties, drug likeness, synthesizability.

In particular, the confirmed part A is acid group (COOH), ester groups (COOR) or amide groups (COONHR), meanwhile the combined part B is elongated chain as alkyl group (CH₂-CH₂, CH₂-CH₂-CH₂), ether group (CH₂-O) or amine group (CH₂-N).

For combination BC, the experimental/historical data, parameters bc need to be considered is in range of target bioactivity/selectivity, ADME properties, drug likeness, synthesizability. In particular, part B is the same as in combination AB, part C can be selected from long chain substitution groups. This requirement is closely concerned to the synthesizability.

For combination CD, the experimental/historical data, parameters cd need to be considered is in range of target bioselectivity, ADME properties, drug likeness, synthesizability. part C is the same as in combination BC, and part D should be focused on the saturated or unsaturated ring like structure to avoid single substitution group.

Taking together of the two considerations, part A needed to be considered was acid group (COOH), ester groups (COOR), or amide groups (COONHR), part B was alkyl groups (CH₂-CH₂, or CH₂-CH₂-CH₂), ether groups (CH₂-O) or amine groups (CH₂-N), part C was long chain substitution groups, and part D was saturated or unsaturated mono-cyclic rings or bi-cyclic structures.
7) A ccording to the results of comparative analysis of structures of experimental/historical data, the tertiary consideration of multidimensional matrix was the following:
   For combination ABC, the experimental/historical data, parameters abc need to be considered is in range of target selectivity, ADME properties, toxic side effects, drug likeness, synthesizability. It can be confirmed that A = COOH or COOR; B = CH₂ or CH₂-CH₂; C = CH₃ or long chain substitution groups.

For combination BCD, the experimental/historical data, parameters bcd need to be considered is in range of target selectivity, ADME properties, drug likeness, synthesizability, It can be confirmed that B = CH₂ or CH₂-CH₂; C = CH₃ or long chain substitution groups, D = 5-5 bi-cyclic rings, 6-5 bi-cyclic rings, and 5 membered rings in part D can be considered as an opened rings.
8) According to the results of comparative analysis of structures of experimental/historical data, the fourth consideration of multidimensional matrix was the following:
For combination ABCD, the experimental/historical data, parameters abcd need to be considered is in range of target bioactivity/selectivity, toxic side effects, ADME properties, drug likeness, synthesizability. ABCD can be separated as:
   A = COOH, COOR; B = CH₂-CH₂; C = CH₃ or long chain substitution group; D = 5 membered mono-cyclic rings, 5-5 bi-cyclic rings, 6-5 bi-cyclic rings, 5 membered opened rings, and the combination thereof.

9) According to the results of comparative analysis of experimental/historical data, the structure of compound can be confirmed in particular as the following, see table 1.

**Table 1 Structural optimization of Captopril related compounds**

| No. | Name of compound | Structure of compounds | Modification of compound structures |
|---|---|---|---|
| 1 | Enalapril | 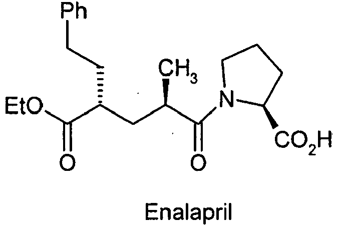 | A=COOEt |
| | | | B=CH₂-CH-CH₂CH₂Ph |
| | | | C=CH₃ |
| | | | D=5 membered mono-cyclic rings |
| 2 | Lisinopril | 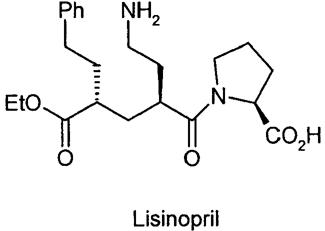 | A=COOEt |
| | | | B=CH₂-CHCH₂CH₂Ph |
| | | | C=CH₂CH₂NH₂ |
| | | | D=5 membered mono-cyclic rings |
| 3 | Ramipril | 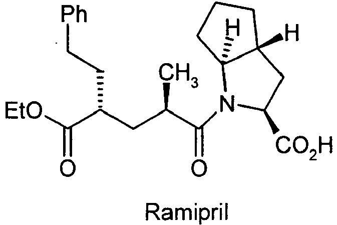 | A=COOEt |
| | | | B= CH₂-CHCH₂CH₂Ph |
| | | | C=CH₃ |
| | | | D=5-5 bi-cyclic rings |
| 4 | Trandolapril | 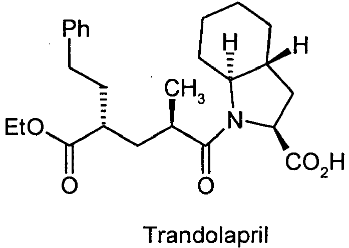 | A=COOEt |
| | | | B=CH₂-CHCH₂CH₂Ph |
| | | | C=CH₃ |
| | | | D=saturated 6-5 bi-cyclic ring |
| 5 | Quinapril Moexipril | 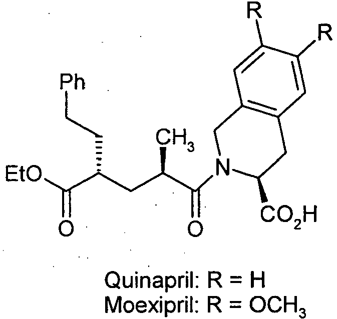 | A=COOEt |
| | | | B= CH₂-CHCH₂CH₂Ph |
| | | | C=CH₃ |
| | | | D=unsaturated 6-5 bi-cyclic ring |
| 6 | Benazepril | 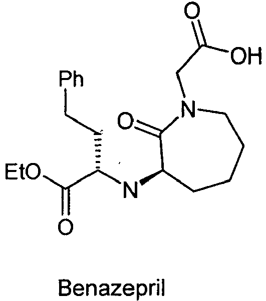 | A = COOEt |
| | | | B = CH₂-CHCH₂CH₂Ph |
| | | | C = combination of the opened and closed ring, consistence in stereochemistry |
| | | | D= opened 5 membered mono-cyclic ring, recombined as ring with the methyl group in position C |

### Example 2 Structural optimization of captopril serials compound

The same steps as Example 1 were carried out after the confirmation of Captopril, Enalapril, Lisinopril, Ramipril, Trandolapril, Quinapril, Meocipril, Prindopril, Benazepril, Fosinopril.

As for efficacy, Trandolapril, Ramipril, Prindopril and Meocipril have shown strong efficacy, Studies of compound structures by using multidimensional matrix and in reference to the comparison with the experimental/historical parameters, part D has determinative effect on the bioactivity and selectivity of the compounds. To prevent the metabolism of the five membered rings of part D is the main factor to improve compound efficacy. The saturated ring (such as Trandolapril, Ramipril, Prindopril) compared to the aromatic rings (Meocipril and Quinapril) has even stronger effects. In the field of toxicity and side effects, Ramipril shows the best toxicity and side effects profiles, indicating the saturated ring below five membered rings will be the best choice.

Lisinopril, Fosinopril, Benazepril and Quinapril show relatively weaker efficacy and toxicity and side effects profiles, indicating the importance of part C and part D, and the small and simple substitution group in part C will be the best choice.

Prindopril exhibits the advantages of functional group substitution in bio-equivalence. In combination with the compound type of structural comparison, according to molecular design by multidimensional matrix, design of compound structures can be optimized, the prerequisite for the optimization is to ensure the bioactivity of compound maintained below 10µM, and keep and optimize its bioselectivity, The protocol is in Table 2.

**Table 2 Structural optimization of Captopril related compounds**

| No. | General formula of compound structure | Options of modifiable part |
|---|---|---|
| 1 | 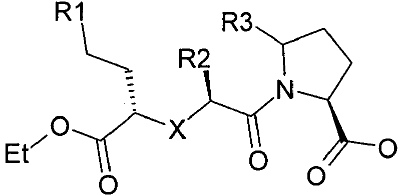 | R1=phenyl-ring, thiazol- |
| | | R2=cyclopropyl, CF₃ |
| | | R3=F, F₂, CH₃, CF₃ |

### Example 3 Structural optimization of Pioglitazone related compounds

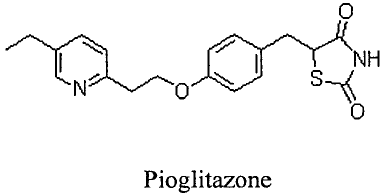

**Table 3 Partitioning the structure of pioglitazone and its modifiable part**

| Structural partitioning of Pioglitazone | Modifiable part of Pioglitazone |
|---|---|
| 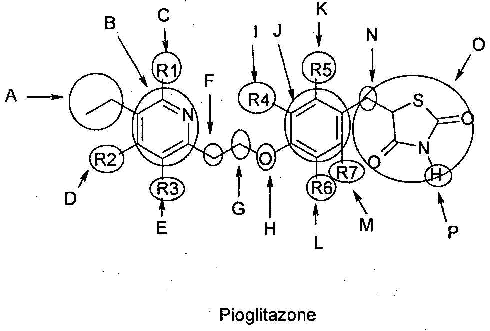 | 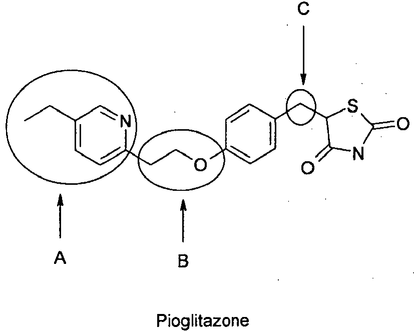 |

### (I) Structure partitioning of Pioglitazone

According to the structural type of Pioglitazone, it can be divided into 16 parts as A, B, C, D, E, F, G, H, I, J, K, L, M, N, O and P by building blocks, as listed in Table 3.

Part J, O, P determine and affect bioactivity/cell activity of the compound, should belong to the un-modifiable part.

Part N belongs to the partial affect bioactivity/cell activity, and it also affects bioselectivity of the compound at certain degrees. The proper medication to it can adjust bioselectivity, and need to be considered in combination with G, H, K. It thus is considered entirely during the optimization of the compound structure during the design of drug candidates.

I, K, L, M belong to the substitution groups or functional groups, which are the not-to-consider parts.

Part F, G, H belong to the connection/linker part, can adjust the modifiable part or changeable part for bioselectivity, ADME properties, toxicity and side effects, drug likeness and synthesizability, and to be considered entirely.

Part A and B belong to the parts are able to adjust bioselectivity, toxicity and side effects, ADME properties, drug likeness and synthesizability, which can be confirmed as modifiable part or changeable parts. C, D, E belong to substitution groups or functional groups, which are not-to-consider parts and can be considered entirely with part A, B, C, D, and E.

Based on the results of molecular structure analysis, the modifiable parts of Pioglitazone are A, B, C, as in Table 3.

Most modification of part A is to change the heterocyclic structures, such as phenyl rings and other heterocyclic structures, but mostly is pyridine rings with the substitution groups.

The majority of considerations for modification of part B are the connecting/linker function, so the length of carbon chain and the bioequivalent substitution of C, O, N is the major modification.

Part C is are more complicated part, modific ation of this part would affect bioactivity/cell activity, and should be not changed if possible. The major modification can be the length of carbon chain and bioequivalent substitution of C, O, N.

Meanwhile, for part A, the experimental data parameter a needed to be considered is in range of bioselectivity, toxicity & side effects, ADME properties, drug likeness and synthesizability.

For part B, the experimental data parameter b needed to be considered is in range of the factors that affect bioselectivity, toxicity & side effects, ADME properties and synthesizability of the compound.

For part C, the experimental data parameter c needed to be considered is in range of bioactivity, factors that adjust bioselectivity, toxicity & side effects, ADME properties, drug likeness and synthesizability.

According to the results of comparative analysis of the experimental data, the preferred representative compound structures are the following:
By comparative analysis of matrix Aa, it was confirmed that the typical structures for part A was pyridine rings.

By comparative analysis of matrix Bb, it was confirmed that the typical structures for part B was bioequivalent substitution of C, O, N.

By comparative analysis of matrix Cc, it was confirmed that the typical structures for part C was the length of carbon chain and bioequivalent substitution of C, O, N.

Taking together all the abovementioned analysis results, it could be confirmed that A could be pyridine ring, part B could be -NCH₃CH₂CH₂O, part C was kept. The following structure could be confirmed quickly as:

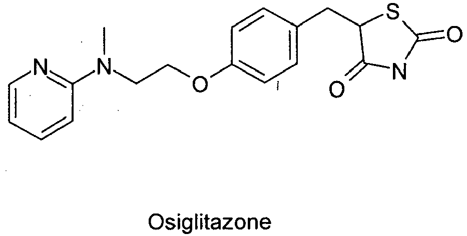

### Example 4 Structure optimization of Omeprazole related compounds

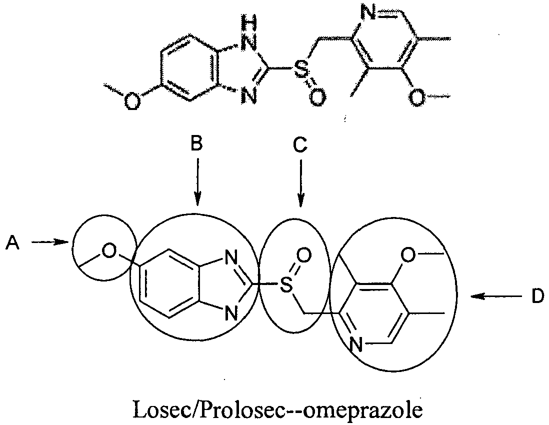

1) Partition Omeprazole by the building block structures, which can be classified as four parts of A, B, C, D;
2) According to experimental data or literatures, the numbers of possible combinations of part A, B, C, D in multidimensional matrix is not less than ten thousands. According to variable factors that affect drug candidates to determine the modifiable parts of drug candidates. The particular steps are listed as below:
   ① According to experienced/histtorical data, the effective functional groups to replace methoxy group in part A comprise R, Ar, RO, RN, RS, RCO, RCON, etc., and can be located at position 1 and 2.
   ② For part B, according to analysis of databases, effective functional groups to substitute part B comprise 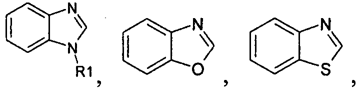 etc..
   ③ For part C, according to analysis of databases, effective functional groups to substitute part C comprise SON, SO₂N, SO₂C, SC, etc.
   ④ For part D, according to analysis of databases, effective functional groups to substitute part D comprise Ar or 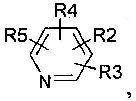 wherein, R₂, R₃, R₄ and R₅ can be R,OR, etc, respctively.
3) According to experimental data, selected variable factors and the variables that affect drug. The particular steps are listed as below:
   According the analysis of the molecular structures for the same target, structural analysis of biological target, and analysis of experimental databases etc., part B and part C belong to the necessary structural parts for bioactivity, and are unmodifiable part. Wherein, N-substituted indol rings in part B can result in the substitution groups instable in acidic condition and metabolism process. H connected with N in indol has important fucntions for drug bioactivity and the pH property of the compound. Although it is reasonable in aspect of bio-equivalence, but the substitution of N by O or S is not rational. Mono-oxidated S in part C is the very important bioactivity moiety, the conversion to be bis-oxidated S is not good for bioactivity. Although it is reasonable in aspect of bioequivalence to replace C that connects pyridine ring and S to be N, it is not rational for "drug likeness"and specificity of compound.

By using multidimensional matrix to analyze structures, the focus of molecular design is on part A and part D. In reference to commercial compound databases and experimental data, use multidimensional matrix to arrange, combine, analyze, and optimize the structures of the core part A, D. Firstly, classify and exclude the structures and substitution positions of the certain types of molecular structures, select the minimum factors, then conduct synthesis test to find out the best substitution groups and positions for part A, aromatic rings and the substitution groups and position for part D. According to compound synthesis databases, verify the synthesis of the new compound structures.

For part A, the major concerns are the selection of phenyl and heterocyclic rings and the positions and types of the substitution groups, and the bio-equivalence, pharmacokinetics & pharmacology, natural products, syntheziable property and compound pH property, etc. Multidimensional matrix can be untilized for the combination of C, O, N, S and halogen to find out the rational options.

For part D, the key is the selection of aromatic rings, and the possibility is excessive. For the certian compound structures, because the molecular structures with the similar structure-activity always use 2- substitution of pyridine, the necessary strategy for design is to utilize different N-contained heterocyclic structures. Permutaions of molecular multidimensional matrix provide many possibilities for substitutions. The molecular structure analysis using multidimensional matrix is focused on the 2 position substituted pyridine ring to improve and enhance the bioactivity, etc. For substitution groups on pyridine ring, the main aspects are pH property of compound, pharmacokinetics & pharmacology, bio-equivalence, "drug likeness", natural products, etc.
4) According to the results of structural comparison of each structure part and the experimental data sequence, the representative compound structural types were selected out, in particular as the following:
   By comparative analysis of matrix Aa, it was confirmed that the typical structures for part A were R, Ar, RO, RN, RS, RCO, RCON, etc., and it could be located at position 1- and 2-.

By comparative analysis of matrix Bb, it was confirmed that the typical structures for part B were benzopyrazole.

By comparative analysis of matrix Cc, it was confirmed that the typical structures for part C were -SOCH₂-.

By comparative analysis of matrix Dd, it was confirmed that the typical structures for part D were 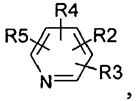 Ar, etc.
5) According to resutls of comparative analysis of the experimental data, the considerations of multidimensional matrix are as below:
For combination ABCD, the experimental data parameter abcd needed to be considered is in range of bioactivity/selectivity, toxicity & side effects, ADME properties, drug likeness, synthesizability, which comprise:
   A= OR, R, R can be H, alkyl or substituted alkyl, particularly halogenated alkyl;
   B= benzopyrazole; C= -SOCH₂-; 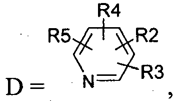 wherein, R₂, R₃, R₄ and R₅ can be R,OR, etc., R can be H, alkyl or substituted alkyl , particularly halogenated alkyl or alkoxyalkyl.

(6) According to comparative analysis results with the experimental data, the particular compound structures were confirmed as the following, as Table 4.

**Table 4 Structural optimization of Omeprazole serials compounds**

| No. | Name of compound | General formula of compound structures |
|---|---|---|
| 1 | Lansoprazole Takepron | 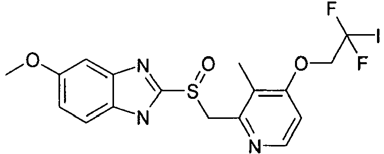 |
| 2 | Pantoprazole Protonix | 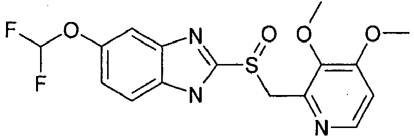 |
| 3 | Rabeprazole Pariet | 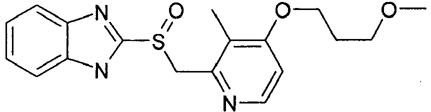 |
| 4 | Esomeprazole Nexium (Only considering the chiral) | 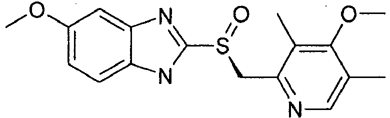 |

### Example 5 Process of structural optimization of Prozac serials compound

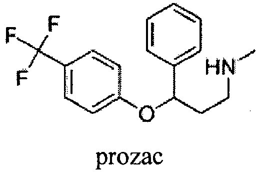

**Table 5 Partitioning the structure of Prozac and its modifiable part**

| Structure partitioning of Prozac | Modifiable parts of Prozac |
|---|---|
| 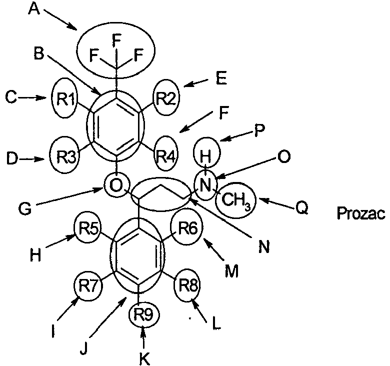 | 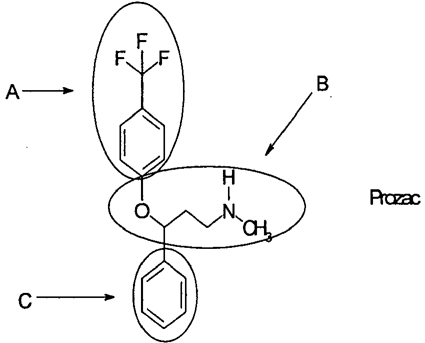 |

(1) Partition the structure of Prozac by compound building blocks into 17 parts as part A-Q, see Table 5. If considering all the 17 parts, the number of compounds by permutation and combination is tremendous.
(2) According to the structures that affect bioactivity/cell activity of drug candidates, un-modifiable part, not-to-consider part, and modifiable part was determined. The particular steps are listed as below:
   Part G, N, O have closed connection with target bioactivity/cell activity of the compound, belong to core structure. In particular, parts of N, O, G are the parts should not easily changed or modified, but part N can be replaced by bio-equivalent functional group. Part P, Q are also the parts should not easily changed or modified in consideration of target bioactivity/cell activity, but the hydrogen bond donor function before or after metabolism should be considered. Thus, part G, N, O, P, Q are classified as one part for consideration.
   Part A, B, C, D, E, F belong to substitution group part, have effects on toxicity & side effects of the compound. They can be not considered in the early stage in design, and considered entirely after structure optimization.
(3) According to results of structural analysis and confirmation of the target compound, A, B, C are determined as modifiable part (Table 5).
(4) According to experimental data, select the variable factors and the variables that affect the drug. The particular steps are listed as below:
   For part A, it is a reasonable modification to keep phenyl ring structures and change the substitution groups.

For part B, considering the history for drugs in central nervous system, early drugs belong to MAOI serials compound structure types, tri-cyclic compound types. The following structures can be confirmed as: CH₂CH₂N(CH₃)₂, CH₂CH₂NHCH₃, CH₂CH₂CH₂N(CH₃)₂ and CH₂CH₂CH₂NHCH₃. O atom is considered as an equivalent group to connect functional groups.

For part C, keeping phenyl ring structure is the prior factors that must be considered, reasonable modification is to change the substitution groups.

According to experimental databases, variable factor a for part A needs to consider bioselectivity, toxicity & side effects, ADME properties, drug likeness and synthesizability. Substitution groups for this part should be considered.

According to experimental databases, variable factor b for part B needs to consider ADME, bioselectivity, cell activity, metabolism, toxicity & side effects, drug likeness, synthesizability. In particular, number of the rotatable bonds for drug likeness and bioselectivity, toxicity & side effects, etc., C atom is preliminary considered as the equivalent group of O atom. In consideration of the characteristics of natural products, O atom will be kept preferentially. Ring-like compound structures are also a modifiable parts.

According to experimental database, variable factor c for part C needs to consider factors of bioselectivity, toxicity & side effects, ADME properties, drug likeness, synthesizability, etc., Substitution groups for this part can be considered. It is preferentially to consider the substitution groups of part A.
(5) According to the results of structural comparison of each structural part and the experimental data sequences, the representative compound structure types were selected out, in particular as the following:
   By comparative analysis of matrix Aa, it was confirmed that the typical structures for part A were substituted phenyl rings (such as halogen or CN substituted phenyl ring, wherein, CN is equivalent to halogen), five membered ring di-ether substitued phenyl ring structural types, oxygen-containing five membered heterocyclic, which are common in natural products;

By comparative analysis of matrix Bb, it was confirmed that the typical structures for part B was N atom-containing ring-like structures, preferentially six membered ring structures; CH₂CH₂N(CH₃)₂, CH₂CH₂NHCH₃, CH₂CH₂CH₂N(CH₃)₂ and CH₂CH₂CH₂NHCH₃, or equivalent groups of O atom such as C or N.

By comparative analysis of matrix Cc, it was confirmed that the typical structures for part C were the stereochemical configuration types which were introduced to beta-position related to O atom, five membered heterocyclic introduced with phenyl ring or the substituted phenyl rings by substitution groups, preferentially halogen, more preferably F atom.
(6) According to resutls of comparative analysis of the experimental data, the considerations of multidimensional matrix are as below:
For combination ABC, the experimental data parameter abc needed to be considered is in range of target bioactivity/selectivity, toxicity & side effects, ADME properties, drug likeness, synthesizability, which can be specified as:
   A = five membered ring diether, halogen substituted phenyl ring, oxygen-containing five membered heterocyclic; B = CH₂CH₂NHCH₃, CH₂CH₂N(CH₃)₂, CH₂CH₂CH₂N(CH₃)₂ or stereo structures; C = phenyl rings, substituted phenyl ring , such as F or Cl substituted phenyl rings.

(7) According to comparative analysis results with the experimental data, the particular compound structures were confirmed as the following, as Table 6.

**Table 6 Structural optimization of Prozac serials compound**

| No. | Name of compounds | General formula of compound structures |
|---|---|---|
| 1 | Paroxetine | 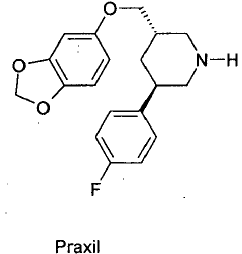 |
| 2 | Zoloft | 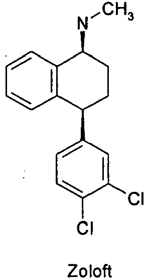 |
| 3 | Citalopram | 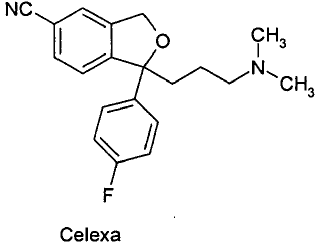 |
| 4 | Lexapro | 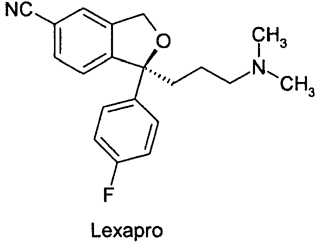 |

### Example 6 Structural optimization of analogue of Gefitinib

Gefitinib (Irresa, Gefinib, ZD1839) is a selective tyrosine kinase inhibitor for Epidermal Growth Factor Receptor (EGFR), as the new anticancer drugs, with its structure as the following formula:

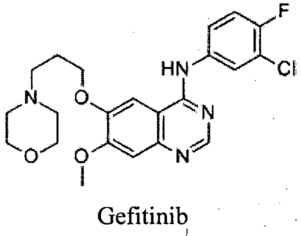

**Table 7 Structure partitioning and modifiable part of Gefitinib**

| Structure partitioning of Gefitinib | Modifiable part of Gefitinib |
|---|---|
| 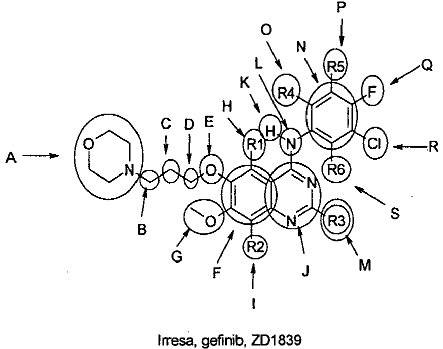 | 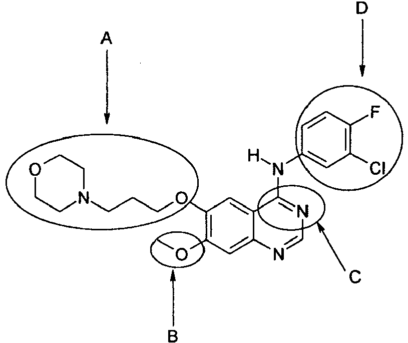 |

### (I) Partitioning of compound structure and determination of structures

Gefitinib can be classified into 19 parts by building blocks as part A, B, C, D, E, F, G, H, I, J, K, L, M, N, O, P, Q, R and S, as the left column of Table 7.

This compound structure type belongs to proof-of-concept for new drug discovery, wherein modifiable parts in the compound structure are rather broad.

Part F, J, K, L, M is closely connected with efficiency of target bioactivity/cell activity of the compound, belong to unmodifiable or unchangeable part , but part J is considered as the part be able to do structural modification, but the bi-cyclic structures should not be easily modified.

Part A, B, C, D, E are modifiable parts, the major factors for consideration are ADME properties, drug likeness, toxicity & side effects, target bioselectivity, synthesizability. Additional possible factor is target bioactivity.

Part G is modifiable part, major factors that must be considered are ADME properties, drug likeness, toxic side effects, target bioselectivity, synthesizability. Additional possible effected factor is target bioactivity.

Part H, I are substitution groups or functional groups, which are factors not-to-consider in the earlier stage in design.

Part N is modifiable part, the major factors to be considered are ADME properties, drug likeness, toxic side effects, target bioselectivity, synthesizability, Additional possible effected factor is target bioactivity. The major structure type need to be considered should be substitution groups.

Part O, P, Q, R, S are substitution groups or functional groups, which are factors not-to-consider in the early stage in design.

After the completion of multidimensional matrix structural analysis and structure determination, the modifiable parts of Gefitinib are determined as A, B, C, D, which are listed in the right column in Table 7.

### (II) Structural optimization of the compound

The major factors to be considered for part A are ADME properties, drug likeness, toxicity & side effects, target bioselectivity, synthesizability. Additional effected factor for consideration is target bioactivity.

The major factors to be considered for part B are ADME properties, drug likeness, toxic side effects, target bioselectivity, synthesizability.

The major factors to be considered for part C are target bioactivity, ADME properties, drug likeness, toxicity & side effects, target bioselectivity, synthesizability.

The major factors to be considered for part D are ADME properties, drug likeness, toxicity & side effects, target bioselectivity, synthesizability. The major factors to be considered are phenyl ring substitution groups or functional groups, mainly as the simple substitution groups, such as halogen, cyano, triple bond or double bond (mainly considering the characteristics of the anticancer drug).

**Table 6 Structural optimization of Gefitinib analogues**

| No. | Factors to be considered | Obtained optimized compound structures |
|---|---|---|
| 1 | ADME properties, drug likeness and synthesizability in Part AB; | 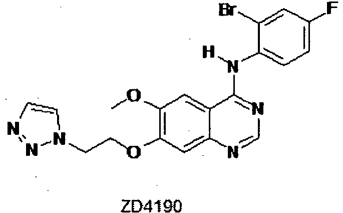 |
| | Maintaining part C ; | |
| | ADME properties, drug likeness and synthesizability in part D, wherein, | |
| | substitution by halogen and phenyl ring are considerable factors (triple bond substitution group can be viewed as to partially compensate target bioactivity) | 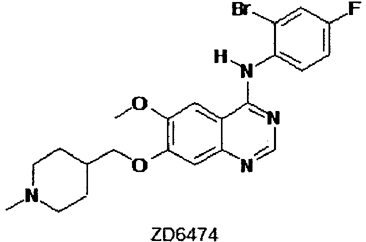 |
| | | 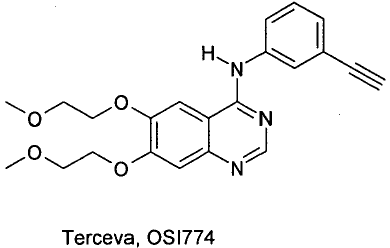 |
| 2 | ADME properties, drug likeness, synthesizability, toxic side effects, target bioselectivity, additional target bioactivity/cell activity (N atom nucleophilic point Micheal acceptor) and synthesizability in part AB, and maintaining part CD | 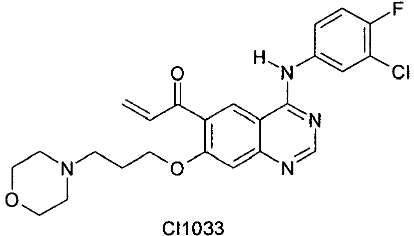 |
| 3 | ADME properties, toxic side effects, target bioselectivity, drug likeness in part AB; | 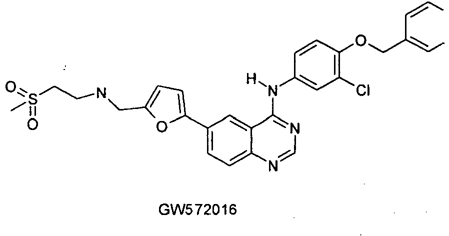 |
| | Maintaining part C; | |
| | ADME properties, drug likeness and synthesizability in part D | |
| 4 | ADME properties, drug likeness, toxic side effects, target bioselectivity, additional target bioactivity, synthesizability in part AB; | 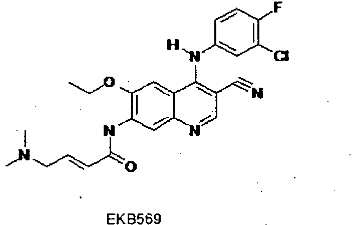 |
| | Toxic side effects, target bioselectivity | |
| | in part C; Part D can be unchanged, or considered for ADME properties, drug likeness, target bioselectivity and the synthesizability | 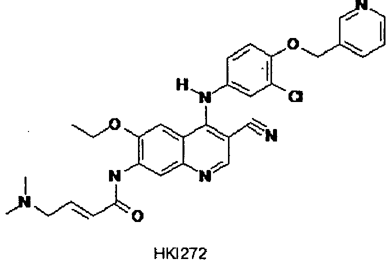 |

### Example 7 Structural optimization of the analogues of Oxazolidinone antibiotics Linezoline

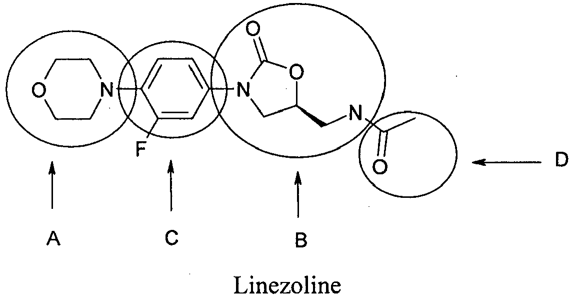

Oxazolidinone antibiotics such as Linezoline has efficacy to many of the stubborn Gram-positive bacteria, which comprise vancomycin-resistant enterococcus feces, methicillin-resistant staphylococcus aureus, penicillin-resistant streptococcus pneumoniae, etc. It may inhibit bacterial protein synthesis in the early transcription of mRNA. Absorption after oral administration is rapid and complete. Many unpublished clinical research data show that Linezolid has efficacy to adult's pneumonia, skin infections, vancomycin-resistant enterococcus feces, etc. The adverse reactions are similar to β amide group antibiotics, clarithromycin, vancomycin, etc. Linezolid is the first approved drug for the treatment of oral antibiotics vancomycin-resistant enterococci. As the oxazolidinone serial drugs have unique mechanism of action and very wide antibacterial spectrum, the treatments of highly resistant Gram-positive bacteria are effective, all these make it extremely valuable drug be able to replace the application of other drugs. Based on this, modification of the structure of Linezoline is to obtain improved compound structures.

The detailed steps are the following :
1) Partition the structure of Linezoline by building blocks, as part A, B, C, D;
2) According to experimental data or literatures, the number of possible combination of part A, B, C, D in multidimensional matrix is not less than ten thousands. According to variable factors that affect drug candidates to determine the modifiable parts of drug candidates. The particular steps are listed as below:
Design of part D: use the simple substitution groups;
   ① part A, the key part to determine the activity of compound, according to experienced data, the changeable structures are saturated heterocyclic or aromatic heterocyclic, saturated heterocyclic is preferred.
   ② part B, the key part to determine the activity of compound, according to analysis of the databases, the replaceable effective functional groups in part B comprises substitution N atom outside of the rings (to eliminate hydrogen bond), O, S, etc.
   ③ part C: according to analysis of databases, the replaceable effective functional groups in part C comprise substituted phenyl rings and aromatic heterocyclic rings.
   ④ part D: adjust DMPK properties, solve the problem in metabolism. According to analysis of databases, the replaceable effective functional groups in part D comprise simple substitution groups.

3) According to experimental data, select variable factors and the variables that affect drug. The particular steps are listed as below:
By using multidimensional matrix to analyze structures, the focus of molecular design is on part A and part B. In reference to commercial compound databases and experimental data, The Multidimensional matrix can be utilized to arrange, comnine, analyze, and optimize the structure of the core part A, B. Firstly, classify and exclude the structures and substitution positions of the certain types of molecular structure, select the minimum factors, then conduct synthesis test to find out the best substitution group and position for part A, aromatic rings and the substitution groups and position for part B. According to compound synthesis databases, verify the synthesis of the new compound structures.

4) According to the results of structural comparison of each structure part with the experimental data sequences, the representative compound structure types were selected out, in particular as the following: 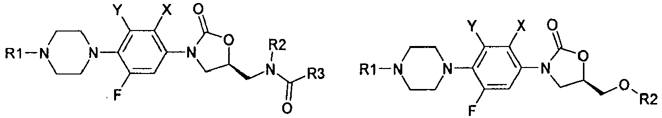 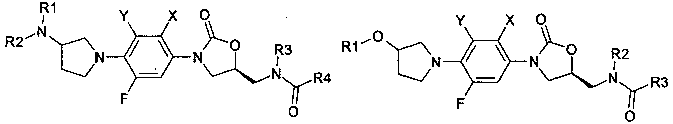 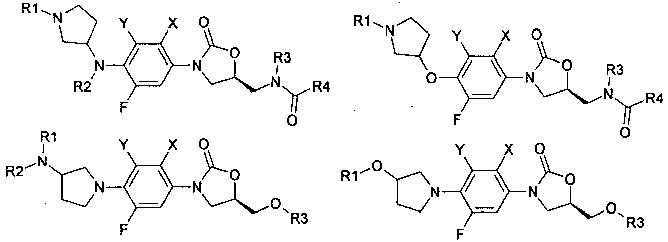
wherein, R1, R2, R3, R4 is any substitution groups, such as H, alkyl, cyclic alkyl, acyl, cyclic acyl, substituted acyl, sulfonamido group, alkyl aminosulfonyl, etc.
X, Y is the conventional substitution groups on aromatic rings, such as H, halogen , alkyl, alkoxy, cyclic alkyl, acyl, etc.

5) According to structural comparative analysis of the experimental data, modify the possible modifiable parts in the first two structures of step 4) and obtain the following structures: 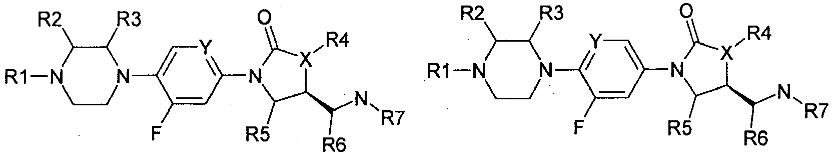
wherein, R1, R2, R3, R4, R5, R6, R7 is any substitution group, such as H, alkyl, cyclic alkyl, acyl, cyclic acyl, substituted acyl, sulfonamido group, alkyl aminosulfonyl, etc. X, Y is CH, NH, O, S;

6) Based on step 5), according to results of the comparative analysis of experimental data, in consideration that R2, R3, R4, R5, R6 have less effects to activity, the following structure formula was confirmed: 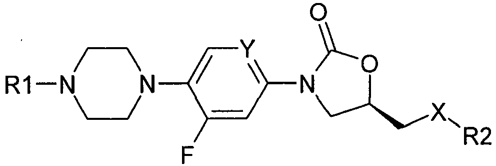 wherein, the definition of each substitution group is listed as below:

| Compound No. | X | Y | R1 | R2 |
|---|---|---|---|---|
| 1 | NH | CH | C(O)CF₃ | C(O)CF₃ |
| 2 | | | HOCH₂C(O) | cyclopropyl |
| 3 | | | C(O) cyclopropyl | C(O)CF₃ |
| 4 | | | cyclopropyl | cyclopropyl |

The protocol of the synthesis of the abovementioned compound is shown as the following:

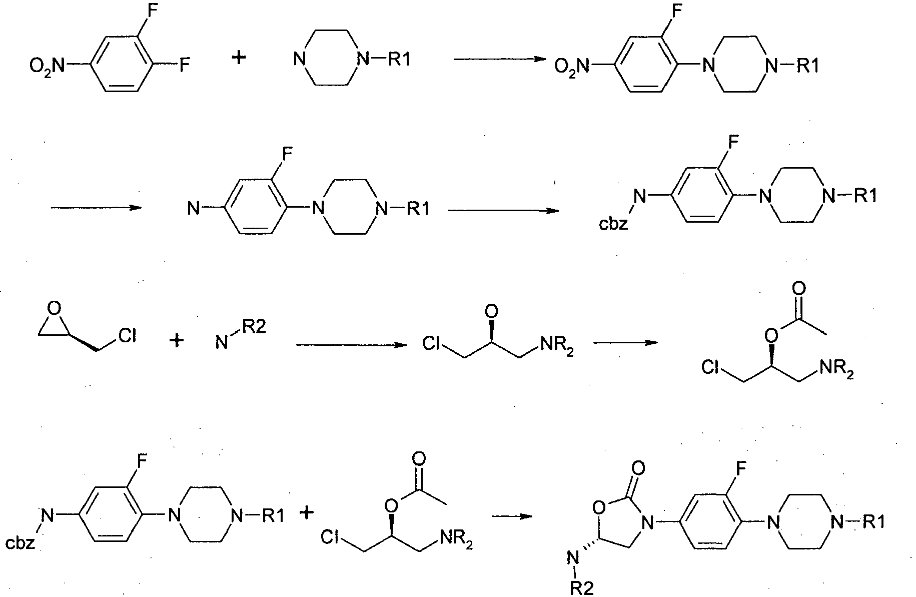

By the abovementioned method, four compounds were obtained and passed the MIC activity detection. These compounds exhibited the similar inhibitory concentration indexes as Linezolid, the results are MIC₅₀ (A) in range of 1-1.5; MIC₅₀ (B) in range of 0.25-0.75.
wherein, antibacterial activity of compound was evaluated by MIC (MIC = minimum inhibition concentration, the lowest drug concentration for reducing growth by 50% or more). Metthicillin-susceptible Staphylococcus was used for A, Penicilin-suscepible Streptococcus pneumonia for B. The steps in experiments were according to the standard detection steps and methods. The unit for inhibitory concentration index is µg /ml.

## Claims

1. A method for optimizing the molecular structure of drug candidate, which comprises the following steps:
(1) Partition the structure of target compound according to basic building blocks, and assign the corresponding structural parts with uppercase letters of A, B, C, D...Y or Z respectively, define the modifiable parts of the drug candidate, select the selectable variables in the modifiable parts respectively, wherein, the variables of modifiable part A are selected from A1, A2, A3...An, the variables of modifiable part B are selected from B1, B2, B3...Bn, the variables of modifiable part C are selected from C1, C2, C3...Cn, the variables of modifiable part D are selected from D1, D2, D3...Dn ......, the variables of modifiable part Y are selected from Y1, Y2, Y3...Yn, the variables of modifiable part Z are selected from Z1, Z2, Z3...Zn, wherein, n is a natural number;
(2) Select variable factors and their variables in reference to the experimental data, wherein the variable factors are represented by lowercase letters of a, b, c, d...y or z, wherein, the variables of variable factor a are selected from a1, a2, a3...an, the variables of variable factor b are selected from b1, b2, b3...bn, the variables of variable factor c are selected from c1, c2, c3...cn, the variables of variable factor d are selected from d1, d2, d3...dn, ......, the variables of variable factor y are selected from y1, y2, y3...yn, the variables of variable factor y are selected from z1, z2, z3...zn, wherein, n is a natural number;
(3) By permutation of multidimensional matrix, analyze the corresponding variables of the modifiable part A, B, C, D...Y or Z in step (1) and the corresponding variables of the variable factor a, b, c, d...y or z in step (2), in reference to the results of structural comparison between the structure parts and experimental data, select the preferred representative structure types of compound as A', B', C', D'...Y' or Z', and complete the design and optimization of the structure of the drug candidate.

2. The method of claim 1 comprises the following steps:
(1) Partition the structures of the target compound according to the building blocks;
(2) Determine the structure parts of the drug candidate molecule that affect target bioactivity/cellular activity in reference to the experimental data, and assign them as un-modifiable parts;
(3) Analyze the structure of the target compound and confirm the structures, determine the modifiable parts of the drug candidates, assign the corresponding structure part with uppercase letters of A, B, C, D...Y or Z respectively, select the selectable variables in the modifiable parts respectively, wherein, the variables of the modifiable part A are selected from A1, A2, A3... An, the variables of the modifiable part B are selected from B1, B2, B3...Bn, the variables of the modifiable part C are selected from C1, C2, C3... Cn , the variables of the modifiable part D are selected from D1, D2 , D3...Dn ......, the variables of the modifiable part Y are selected from Y1, Y2, Y3...Yn, the variables of the modifiable part Z are selected from Z1, Z2, Z3...Zn, wherein, n is a natural number;
(4) Select the variable factors and their variables in reference to the experimental data. The variable factors are represented by lowercase letters of a , b , c , d...y or z, wherein, the variables of the variable factor a are selected from a1, a2, a3...an , the variables of the variable factor b are selected from b1, b2, b3 ... bn ,the variables of the variable factor c are selected from c1, c2, c3...cn ,the variables of the variable factor d are selected from d1, d2 , d3...dn , ...... , the variables of the variable factor y are selected from y1, y2, y3...yn , the variables of the variable factor z are selected from z1, z2, z3...zn, wherein, n is a natural number;
(5) By permutation of the multidimensional matrix, analyze the corresponding variables of the modifiable part A, B, C, D...Y or Z in step (3) and the corresponding variables of the variable factor a, b , c , d...y or z, in reference to the results of structural comparison between the structure parts and experimental data, select the preferred representative structure types of compound as A', B', C', D'...Y' or Z'.

3. The method according to claim 1 or 2 further comprises:
when the modifiable parts are defined in step (1) or (3), exclude the not-to-consider part in the modification, the not-to-consider part is selected from any of the substitution groups on the cyclic structures, the functional groups or structure types should not be included in drug-like compounds, or the combination thereof.

4. The method according to any of the claims 1 to 3 further comprises any or all the following steps:
(6) Analyze the structures of the preferred representative compound structure type A', B', C', D'...Y' or Z' selected in step (3) or (5) and confirm the structures. Determine the selectable variables, wherein, the variables of the modifiable part A' are selected from A'1, A'2, A'3...A'n, the variables of the modifiable part B' are selected from B'1, B'2, B'3...B'n, the variables of the modifiable part C' are selected from C'1, C'2, C'3...C'n, the variables of the modifiable part D' are selected from D'1, D'2, D'3...D'n......, the variables of the modifiable part Y' are selected from Y'1, Y'2, Y'3...Y'n, the variables of the modifiable part Z' are selected from Z'1, Z'2, Z'3...Z'n, wherein, n is a natural number;
(7) Select the variable factors and their variables that affect drug candidates in reference to the experimental data, the variable factors are represented by lowercase letters of a' , b' , c' , d'...y' or z', wherein, the variables of the variable factor a' are selected from a'1, a'2, a'3...a'n, the variables of the variable factor b' are selected from b'1, b'2, b'3...b'n, the variables of the variable factor c' are selected from c'1, c'2, c'3...c'n, the variables of the variable factor d' are selected from d'1, d'2, d'3...d'n......, the variables of the variable factor y' are selected from y'1, y'2, y'3...y'n, the variables of the variable factor z' are selected from z'1, z'2, z'3...z'n, wherein, n is a natural number;
(8) By permutation of the multidimensional matrix, analyze the corresponding variables of the preferred representative compound structure A', B', C', D'...Y' or Z' in step (6) and the corresponding variables of the variable factor a', b', c', d'...y' or z' in step (7), in reference to the results of structural comparison between the structure parts and experimental data, select the preferred compound structure type A'B', B'C', C'D'...Y'Z'; or
(9) According to the requirements, based on the methods of step (6)-(8), by analysis of the permutation of multidimensional matrix, select the corresponding variables of the preferred representative compound structure type A'B', B'C', C'D'...Y'Z' and the corresponding variables of the variable factor a'b', b'c', c'd'...y'z', in reference to the results of structural comparison between the structure parts and experimental data, select the preferred representative compound structure type A"B"C", B"C"D"...X"Y"Z"; or
(10) According to the requirements, based on the methods of step (6)-(9), by analysis of the permutation of multidimensional matrix, select the preferred representative compound structure type A"B"C", B"C"D"...X"Y"Z" and the variable factors of a"b"c", b"c"d"...x"y"z", in reference to the results of the structural comparison between the structure parts and experimental data sequences, complete the structure design and optimization of the drug candidate;
(11) Optionally, according to the requirements of the design of drug candidate, repeat part of or all of the above steps by multidimensional matrix to analyze, confirm and optimize the structures of the drug candidate until obtain the desired structure types of drug candidate.

5. The method according to any of the claims 1 to 4, wherein the building blocks comprise any structure unit in molecular structures, which is selected from any of saturated or unsaturated mono-cyclic structure unit, bi-cyclic structure unit, multi-cyclic structure unit, substitution group, functional group or the combination thereof;
wherein, the mono-cyclic structure unit is selected from any of mono-cyclic aromatic ring, mono-cyclic non-aromatic ring, substituted mono-cyclic aromatic ring, substituted mono-cyclic non-aromatic ring or the combination thereof;
the bi-cyclic structure unit is selected from any of bi-cyclic aromatic ring, bi-cyclic non-aromatic ring, substituted bi-cyclic aromatic ring, substituted bi-cyclic non-aromatic ring or the combination thereof;
the multi-cyclic structure unit is selected from any multi-cyclic aromatic ring, multi-cyclic non-aromatic ring, substituted multi-cyclic aromatic ring, substituted multi-cyclic non-aromatic ring or the combination thereof, wherein the number of rings is not less than 3;
the functional group is selected from any of ketone, aldehyde, ester, amine, amide, single bond, double bond, triple bond, halogen, acid, alcohol, thiol, sulfonic acid, phenol, thiophenol or the combination thereof;
the substitution group is structural moiety of any compound, which is selected from any of alkyl group, alkenyl group, alkynyl group, hydroxyl group, ether group, ester group, aryl group, heteroaryl group, cycloalkyl group, heterocyclic group or the combination thereof.

6. The method according to any of claims 1 to 5, wherein the modifiable part refers to the structure part that affects bioactivity or cell specificity of the compound.

7. The method according to any of claims 1 to 6, wherein the experimental data are selected from any of target bioactivity, target bioselectivity, cell activity, toxic side effects, ADME properties, drug likeness, synthesizability or the combination thereof.

8. The method according to any of claims 1 to 7, wherein the experimental data are selected from any of the following database or the combination thereof:
1) database of protein targets commonly used in world drug discovery field and the database of the corresponding compound structure; or
2) database of the structure types of the corresponding compounds for the protein targets commonly used in world drug discovery; or
3) database of core structures for drug discovery; or
4) database of the framework compound for drug molecule; or
5) database of the structure of the verified bioactive compound; or
6) database of the queryable marketed drugs; or
7) database of bioequivalence; or
8) database of the metabolic compounds; or
9) database of the structure of the toxic compound; or
10) database of the active ingredient compound in Chinese medicine; or
11) database of the monomer compound structure of natural products; or
12) database of therapeutics; or
13) database of medical keywords.

9. The application of multidimensional matrix for drug molecule design, wherein, the permutation of the multidimensional matrix is determined jointly by structure factors and experimental data.

10. The application according to claim 9, the drug molecules are selected from any of Me-Too type new drug, drug framework compound, "drug-like" compound, Hit-To-Lead, lead compound or the combination thereof.
